# EUROPEAN PATENT SPECIFICATION

(11) **EP 4 594 753 B1**
(45) Date of publication and mention of the grant of the patent: **19.08.2026**
(21) Application number: 23786648.8
(22) Date of filing: 26.09.2023
(51) Int. Cl.: G01N 33/569, G01N 33/543, G01N 33/58

(54) **SANDWICH IMMUNOASSAY AND KIT FOR DETECTION OF LISTERIA BACTERIA**
SANDWICH-IMMUNOASSAY UND KIT ZUM NACHWEIS VON LISTERIA-BAKTERIEN
IMMUNOESSAI SANDWICH ET KIT POUR LA DÉTECTION DE LA BACTÉRIE LISTERIA

(30) Priority: 27.09.2022 GB 202214119
(43) Date of publication of application: 06.08.2025
(73) Proprietor: University of Strathclyde, Glasgow G1 1XQ (GB)
(72) Inventor: GRAHAM, Duncan, Glasgow G1 1BX (GB); FAULDS, Karen, Glasgow G1 1BX (GB); MAY, Hayleigh, Glasgow G1 1BX (GB)
(74) Representative: Marks & Clerk LLP
(86) International application number: PCT/GB2023/052489
(87) International publication number: WO 2024/069157

(56) References cited:
- WO-A2-2008/116093
- CN-A- 110 470 841
- CN-A- 111 024 938
- YEGENOGLU AKÇINAR HANDE ET AL: "Immunomagnetic separation and Listeriamonocytogenes detection with surface-enhanced Raman scattering", TURKISH JOURNAL OF MEDICAL SCIENCES, vol. 50, no. 4, 23 June 2020 (2020-06-23), pages 1157 - 1167, XP093105868, DOI: 10.3906/sag-2002-234
- CHENG SIYUN ET AL: "An efficient SERS platform for the ultrasensitive detection of Staphylococcus aureus and Listeria monocytogenes via wheat germ agglutinin-modified magnetic SERS substrate and streptavidin/aptamer co-functionalized SERS tags", ANALYTICA CHIMICA ACTA, ELSEVIER, AMSTERDAM, NL, vol. 1187, 7 October 2021 (2021-10-07), XP086852033, ISSN: 0003-2670, [retrieved on 20211007], DOI: 10.1016/J.ACA.2021.339155
- SUH SOO HWAN ET AL: "Nucleic acid aptamers for capture and detection ofListeriaspp", JOURNAL OF BIOTECHNOLOGY, ELSEVIER, AMSTERDAM NL, vol. 167, no. 4, 11 August 2013 (2013-08-11), pages 454 - 461, XP028720832, ISSN: 0168-1656, DOI: 10.1016/J.JBIOTEC.2013.07.027
- BUSCH ROBERT T. ET AL: "Detection and Aggregation of Listeria Monocytogenes Using Polyclonal Antibody Gold-Coated Magnetic Nanoshells Surface-Enhanced Raman Spectroscopy Substrates", FRONTIERS IN NANOTECHNOLOGY, vol. 3, 27 April 2021 (2021-04-27), XP093105870, DOI: 10.3389/fnano.2021.653744
- KEARNS HAYLEIGH ET AL: "SERS Detection of Multiple Antimicrobial-Resistant Pathogens Using Nanosensors", ANALYTICAL CHEMISTRY, vol. 89, no. 23, 5 December 2017 (2017-12-05), US, pages 12666 - 12673, XP093102160, ISSN: 0003-2700, DOI: 10.1021/acs.analchem.7b02653
- WANG JUNPING ET AL: "Rapid detection of Listeria monocytogenes in milk using confocal micro-Raman spectroscopy and chemometric analysis", INTERNATIONAL JOURNAL OF FOOD MICROBIOLOGY, vol. 204, 1 July 2015 (2015-07-01), NL, pages 66 - 74, XP093106100, ISSN: 0168-1605, DOI: 10.1016/j.ijfoodmicro.2015.03.021

## Description

### Field

The present disclosure relates to products and methods for detecting *Listeria* species in an environment.

### Background

The detection of *Listeria* spp. is of particular interest for the food industry and for environmental monitoring. A particular concern is contamination by *Listeria monocytogenes,* which can lead to death in up to 25% of affected individuals.

Up to 10% of humans may be intestinal carriers of *L. monocytogenes* and the bacteria have been found in domestic and feral mammalian species, as well as species of birds, fish and shellfish. The bacteria can be isolated from soil, silage, and other environmental sources. *L*. *monocytogenes* is quite hardy and resists the deleterious effects of freezing, drying, and heat remarkably well for a bacterium that does not form spores. Most *L. monocytogenes* are pathogenic to some degree. The organism has an unusual property of being able to cross the intestinal barrier, the blood-brain barrier and the placental barrier.

In general, other species including *L. innocua, L. welshimeri, L. seeligeri, L. marthii* and *L*. *grayi* are considered nonpathogenic species, although this is not always the case. All *Listeria* species are potential food contaminants; the presence in food of any of these species can be considered to be an indicator of contamination and of the potential presence of *L. monocytogenes,* although being able to determine this accurately is advantageous. Early detection of the presence of *Listeria* is therefore extremely important both from a public health perspective and from an economic perspective.

Traditional culture confirmation methods used in food and environmental safety testing are lengthy. Time-to-result can be greatly reduced by using rapid molecular techniques, but such methods often require skilled technicians and cannot be on-site.

CN111024938 discloses "a rapid detection method for pathogenic microorganisms. The specific steps of the method are: preparing a detection probe, capturing the probe, incubating to obtain a sandwich product, drawing a standard working curve, and detecting a sample to be tested; Among them, the detection probes include nano-gold@nitrogen-doped graphene quantum dot nanomaterials, as well as thiol-containing silent region Raman signal molecules and broad-spectrum recognition molecules modified on the surface of the nanomaterials; the capture probe is a modified antibody of immunomagnetic beads. The Raman detection of pathogenic microorganisms is realized by the combination of detection probes with immunomagnetic beads, that is, the mechanism of specific recognition coupled with broad-spectrum recognition". Akçinar et al. "Immunomagnetic separation and Listeria monocytogenes detection with surface-enhanced Raman scattering." Turkish Journal of Medical Sciences 50.4 (2020): 1157-1167 discloses "a rapid method to enumerate Listeria monocytogenes (L. monocytogenes) utilizing magnetic nanoparticle based preconcentration and surface-enhanced Raman spectroscopy measurements". Cheng et al. "An efficient SERS platform for the ultrasensitive detection of Staphylococcus aureus and Listeria monocytogenes via wheat germ agglutinin-modified magnetic SERS substrate and streptavidin/aptamer co-functionalized SERS tags." Analytica Chimica Acta 1187 (2021): 339155 discloses "a novel surface-enhanced Raman scattering (SERS)-based analytical technique was proposed to simultaneously detect two highly pathogenic bacteria, namely, Staphylococcus aureus (*S. aureus*) and Listeria monocytogenes (*L. mono*) by using a dual-recognition pattern with wheat germ agglutinin (WGA) and nucleic acid aptamers. WGA was modified onto Fe₃O₄@Au magnetic nanoparticles (MNPs) for the efficient capture of *S*. *aureus* and *L. mono* in complex samples (orange juice, extracts of lettuce, and human urine) within 15 min. The streptavidin (SA)/aptamers co-functionalized SERS tags were fabricated by covalent attaching two different Raman reporters and SA molecules onto 45 nm Au NPs and then conjugated with two biotin-aptamers that specifically bind to their target bacteria with high affinity and stability".

It is amongst the objects of the present disclosure to obviate and/or mitigate one or more of the aforementioned disadvantages.

### Summary

The present disclosure is based on studies by the investigators to develop a rapid and easily employed optical detection kit, system and/or method for use in the detection of Listeria.

In a first aspect, there is provided a sandwich immunoassay kit for use in detecting and optionally quantifying *Listeria* in a sample in accordance with the claims appended hereto.

The *Listeria* to be detected may be any *Listeria* species, such as *L. monocytogenes, L. aquatica, L. booriae, L. cornellensis, L. costaricensis, L. goaensis, L. fleischmannii, L. floridensis, L. grandensis, L. grayi, L. innocua, L. ivanovii, L. marthii, L. newyorkensis, L. riparia, L. rocourtiae, L. seeligeri, L. thailandensis, L. valentina, L. weihenstephanensis,* and *L. welshimeri.* In one embodiment, the *Listeria* species comprises, or consists of *L*. *monocytogenes.* In one embodiment, more than one *Listeria* species, such as two, three, four, five or more *Listeria* species, may be detected. In one embodiment *L. monocytogenes* may be detected and optionally discerned from one or more further *Listeria* species.

The first surface may be, for example, a wall or surface of a plate (e.g. a microtitre plate), slide, or channel within a cartridge, such as a microfluidic cartridge. The surface can be made from polymers, such as PMMA. Other exemplary substrates include polystyrene, poly(dimethylsiloxane), polyethylene terephthalate, polyethylene, polypropylene, polylactic acid, poly(D,L-lactide-co-glycolide), polycarbonate, cyclic olefin copolymers, silicon, and glass. In a preferred embodiment the first surface is the surface of a magnetic or paramagnetic particle, or nanoparticle.

The first antibody or antigen binding fragment thereof may be immobilised to the first surface by suitable attachment or bonding to the first surface, by way of, for example, passive or physical adsorption, covalent chemical coupling, non-covalent chemical bonding (e.g. biotin-avidin) or a combination of any of the above.

The first surface may be, for example, a wall or surface of a plate (e.g. a microtitre plate), slide, or channel within a cartridge, such as a microfluidic cartridge. The surface can be made from polymers, such as PMMA. Other exemplary substrates include polystyrene, poly(dimethylsiloxane), polyethylene terephthalate, polyethylene, polypropylene, polylactic acid, poly(D,L-lactide-co-glycolide), polycarbonate, cyclic olefin copolymers, silicon, and glass. In a preferred embodiment, the first surface is the surface of a magnetic or paramagnetic particle, or nanoparticle.

The first and/or second antibodies may independently be a whole antibody. For example, said antibody may be an IgG, IgA, IgE or IgM or any of the isotype sub-classes, particularly IgG1 and IgG4. Said antibodies may be monoclonal and/or polyclonal antibodies. In another teaching, the first and/or second antibodies may independently be an antibody fragment. The antibody fragment may include any polypeptide or protein comprising an antibody antigenbinding site, including Fab, Fab2, Fab3, diabodies, triabodies, tetrabodies, minibodies and single-domain antibodies, including nanobodies. Antibody molecules and methods for their construction and use are described, in for example Holliger & Hudson, Nature Biotechnology 23(9):1126-1136 (2005).

In one embodiment, the first *Listeria* antibody or antibody fragment is capable of binding any *Listeria* species and the second *Listeria* antibody or antibody fragment is capable of binding specific *Listeria* species, such as *L. monocytogenes.* In this manner, the first *Listeria* antibody or antibody fragment is capable of binding any and all *Listeria* species, which may be present in a sample and the second *Listeria* antibody or antibody fragment is capable of binding and enabling detection of specific species. In one embodiment, more than one second *Listeria* specific antibody or antibody binding fragment may be provided. A first of said second *Listeria* specific antibody or antibody binding fragment may be capable of binding any *Listeria* spp and a second of said second *Listeria* specific antibody or antibody binding fragment may be specific for a particular species. In this way, a positive signal will be obtained if any *Listeria* is present in a sample and a further positive signal, which is discernible from the first signal may indicate if the particular species is present in the sample. This may also allow quantification of the relative amounts of total *Listeria* in a sample and the specific species.

Antibodies may be obtained from commercial sources, or using techniques which are standard in the art. Methods of producing antibodies include immunizing a mammal (e.g. mouse, rat, rabbit, horse, goat, sheep or monkey) with one or more *Listeria* spp. of interest in order to raise antibodies thereto. Antibodies may be obtained from immunised animals using any of a variety of techniques known in the art, and screened, preferably using binding of the antibody to *Listeria* spp. of interest.

In an alternative embodiment, the antibody or antibodies used for *Listeria* detection as disclosed herein may include lectins, such as wheat germ agglutinin or Concanavalin A, capable of binding one or more *Listeria* species. In some embodiments, the abovementioned antibody or antibody fragment thereof may be used in conjunction with one or more lectins for detection of *Listeria* in a sample and the specific species. In some embodiments, a DNA aptamer may be used in conjunction with one or more *Listeria* antibody or antibody fragment described above and/or with one or more lectins capable of binding *Listeria.* DNA aptamers are well known in the art and are generally taught in, for example, Mayer, NUCLEIC ACID AND PEPTIDE APTAMERS: METHODS AND PROTOCOLS 535, Humana Press (2009) and S. D. Jayasena, Clinical Chemistry, 1999, 45 (9), 1628-1650.

The magnetic or paramagnetic particles, which comprise the first antibody, or antigen binding fragment thereof immobilised thereto, may simply be deposited in a well, on a substrate, or within a channel of the cartridge, for example. Upon contact with a sample fluid, the antibody/fragment bound magnetic particles are resuspended by the sample fluid and come into contact with any *Listeria,* which may be present in the sample fluid. Alternatively, the antibody/fragment bound magnetic or paramagnetic particles, may be present in a fluid, to which a sample is contacted. For example, a sample may be obtained by use of a swab or the like, which is contacted with the fluid, in order that any *Listeria* bacteria are able to be transferred into the fluid and thereafter are able to bind to the first antibody immobilised to the surface of the magnetic/paramagnetic particles.

As disclosed herein, said magnetic particles preferably have a size in the interval of about 5 nm to about 5000 nm, and more preferably about 50 to about 500 nm. Suitable magnetic particles are known from the literature, and may be synthesised as described herein, for example, or available from commercial suppliers; e.g., Chemicell GmbH, of Berlin, Germany; Bioclone Inc., of San Diego, CA, USA; Nanostructured & Amorphous Materials, Inc., of Huston, TX, USA. Suitable magnetic particles comprise one or more magnetic cores, optionally with a coating matrix of polymers, metal (e.g. gold, silver), silica or hydroxyapatite with terminal functionalized groups. The magnetic core generally consists either of magnetite (Fe₃O₄) or maghemite (gamma Fe₂O₃) with superparamagnetic or ferromagnetic properties. Alternatively, magnetic cores made with magnetic ferrites, such as cobalt ferrite or manganese ferrite can also be produced. Magnetic particles can also be manufactured to order, and given the desired properties for any given application.

The particle or bead to which the second antibody is immobilised may be any suitable particle or bead, such as a latex or non-magnetic metal particle, for example. The second antibody or antigen fragment thereof may be immobilised to the particle or bead surface by suitable attachment or bonding to the surface of the particle or bead, by way of, for example, physical adsorption, covalent chemical coupling, non-covalent chemical bonding (e.g. biotin-avidin) or a combination of any of the above.

Suitable SERS or Raman labels or dyes include 4-(1H-pyrazol-4-yl)pyridine (PPY), 1,2-bis(4-pyridyl)ethylene (BPE), 4,4-dipyridyl (DIPY), 5-(pyridine-4-yl)-1,3,4-oxadiazole-2-thiol (PYOT), 4,4-azopyridine (AZPY), 4-mercaptopyridine (MPY), 4-mrcaptobenzoic acid (MBA), 4-nitrobenzenethiol (4-NBT), Thiophenol, Malachite green isothiocyanate (MGITC), 7(Dimethylamino)-4-methyl coumarin-3-isothiocyanate (DACITC), Rhodamine B isothiocyanate (RBITC), Rhodamine 600 isothiocyanate (XRITC), Fluorescein isothiocyanate (FITC), 6-Carboxyfluorescein (FAM), 5-Carboxytetramethylrhodamine (TAMRA), 1,1-diethyl-2,2-cyanine iodide (DCI), 3,3-diethylthiacarbocyanine iodide (DTCI), Boron-dipyromethene core (BODIPY) dyes such as Cy3, Cy5, for example. In one embodiment, a nanoparticle may include a metal core and a SERS or Raman label or dye surrounding the core or coated on or at the surface of the core. A further coating may surround the core and SERS/Raman label/dye. Electromagnetic waves (such as visible and invisible light waves) can pass through the coating. The coating may also aid in attaching the second *Listeria* specific antibody, to the nanoparticle. Suitable coatings include, but are not limited to, silica, polymers, polyethylene glycol, poly(vinylpyrrolidone (PVP), polyacrylamide (PAM), poly(ethyleneimine (PEI), thioglucose, dextran, for example.

The core may comprise any material known in the art to enhance Raman scattering, wherein the core is preferably a metal core can be made of any metal that provides for enhanced Raman scattering. Suitable SERS active surfaces are known to the skilled addressee, such as described in "Principles of Surface-Enhanced Raman Spectroscopy", Chapter 7 (2009) Authors Le Ru and Etchegoin; G. McNay, D. Eustace, W. E. Smith, K. Faulds, D. Graham, Applied Spectroscopy, 2011, 65, 825-837; and Y. Wang, S. Schlücker, Analyst, 2013, 138, 2224-2238.

The metal may be selected from gold (Au), silver (Ag), copper (Cu), sodium (Na), potassium (K), chromium (Cr), aluminum (Al), lithium (Li), or a combination or alloy thereof. In some embodiments, the metal core can be pure metal or a metal alloy and can be overlaid with at least one metal shell, such as comprised of Au or Ag, for example. In a preferred embodiment, the core is comprised of gold or silver. The nanoparticles can be any shape and hence do not necessary have to be spherical in nature. Thus, rods, stars, cubes, raspberries, hollow (e.g. hollow nanoshells) or any other shape may also be envisaged.

The metal core can be chosen so as to maximize the Raman signal's intensity from the SERS/Raman detectable label/dye. Typically, the metal core has an average size of 20-100nm, such as 30 - 70nm, 40 - 60nm, or around 50nm, for example. Optionally, the metal core may comprise one or more nanoparticles. In such examples wherein the metal core comprises several nanoparticles, such as Si capped Au nanoparticles, the metal core may have an average size of 100-150nm.

As mentioned above, antibodies or antibody fragments may be immobilised to the surface of the aforementioned described particles/nanoparticles using a variety of techniques, including passive adsorption. Usually, passive adsorption techniques employ acidic conditions, but the inventors have found that such acidic conditions are not suitable for use in adsorption of *L*. *monocytogenes* specific antibodies according to the present invention. Thus, in accordance with the present invention, when passively adsorbing *L. monocytogenes* antibodies or antibody fragments to the surface of the aforementioned described particles/nanoparticles, optionally silica coated, a neutral or alkaline pH should be employed. Typically an alkaline pH may be employed, such as pH 8 - 11, such as pH 8.5 - 10, especially pH 9 ± 0.25. Any suitable buffer may be employed for adsorption, such as PB, HEPES, or borate buffer. As well as carrying out the adsorption at the neutral or alkaline pH, an assay/method for use in detecting *L. monocytogenes,* should also be carried out at a neutral or alkaline pH, as defined and preferably in PB (for neutral) or borate(for alkaline) buffer, to ensure nanoparticle stability.

In accordance with the present teaching, significant enhancements in Raman signal can be observed. For example, a Raman signal enhancement of at least 10², for example at least 10³, 10⁴, 10⁵, 10⁶, 10⁸ 10¹⁰, 10¹² or even higher, for one or more labels, when bound in close proximity to the surface of a nanoparticle of the present invention, may be observed. As would be readily apparent to one of ordinary skill in the art, Raman enhancement represents the degree of signal amplification that can be achieved during detection of a particular material. For example, Raman enhancements may allow a vibrational spectrum, or the chemical fingerprint, of low concentrations of *Listeria* to be detected, as described herein.

In accordance with the teaching provided herein, it may be possible to detect *Listeria* that is present at a concentration of ≤ 1000, 100, 50, 25, 10 or 5 colony forming units per ml of sample tested. Typical signal enhancement, as compared to a control sample without *Listeria* present, may be at least 5 times, such as at least 7, 9, 11 or 13 times above background/control.

In a further embodiment, the relative signal intensity (e.g. relative peak height at one or more wavelengths) of Raman signal may be used in a quantitative or semi-quantitative fashion, in order to allow a user to ascertain the concentration of bacteria in a specific sample. This may be important where a low level of bacterial in a particular sample or environment may be accepted and/or tolerated. Thus, a bacterial concentration threshold value may be important and the relative signal intensity may allow a user to determine whether or not the concentration of bacteria is above or below a particular threshold level.

This surface enhancement effect in Raman scattering is well known in the art

Briefly, Raman scattering occurs when a light source irradiates a sample and scattered light is given off. Most of the light is scattered with the same frequency as that of the incident light but a weak component is scattered one vibrational unit different. The weak component is Raman scattering. By subtracting the frequency of the Raman scattered light from the frequency of the incident light, a vibrational spectrum characteristic of the molecule can be obtained. The light can then be detected in a suitable spectrometer, many of which are commercially available.

The detection of Raman scattering is attractive since it uses visible or near infrared radiation to provide the excitation. Moreover, flexible and effective optics can be designed and water gives a weak signal so that detection in aqueous solution is readily possible. Further, the set of signals obtained gives a unique pattern from which a particular label can be identified.

However, the main disadvantage of Raman scattering is that it is not sufficiently sensitive and is not therefore generally suitable for detecting Raman labels at extremely low concentrations, and fluorescence can interfere with detection. The sensitivity of Raman scattering may however be improved. Firstly, if the label is adsorbed onto a suitably roughened metal surface as in accordance with the present teaching, then there is an interaction between the label/dye and the surface electron waves on the metal (plasmons) which provide an enhancement in the intensity of the Raman scattering by a factor claimed to be up to 10⁶. This technique is known as surface enhanced Raman scattering (SERS).

The method for obtaining the Raman or SERS spectrum, may be conventional. However, the following might apply to the spectroscopic measurements. Typically, the methods of the present disclosure may be carried out using incident light from a laser, having a frequency in the visible spectrum ie.380 nm-785 nm, particularly between 400 nm-650 nm (the exact frequency chosen will generally depend on the dye label and on the metal nanoparticle type (i.e. its surface plasmon) used in each case - frequencies in the red area of the visible spectrum, on the whole, may give rise to better surface enhancement effects but this may also depend on excitation wavelength, the metal and the report employed). However, it is possible to envisage situations in which other frequencies, for instance in the ultraviolet (ie. 200 nm-400 nm) or the near-infrared ranges (700 nm-1300 nm) or up to 1600 nm, might be used. Thus, Raman/SERS detection may be conducted between about 200 nm-1550 nm. The selection and, if necessary, tuning of an appropriate light source, with an appropriate frequency and power, will be well within the capabilities of one of ordinary skill in the art, particularly on referring to the available SERS literature. To achieve highly sensitive detection, using SERS, a coherent light source is needed with a frequency at or close to the absorption maximum for the dye. If lower sensitivities are required, the light source need not be coherent or of high intensity and so lamps may be used in combination with a monochromator grating or prism to select an appropriate excitation frequency.

Many devices are suitable for collecting Raman/SERS signals, including wavelength selective mirrors, holographic optical elements for scattered light detection and fibre-optic waveguides. The intensity of a Raman/SERS signal can be measured for example using a charge coupled device (CCD), a silicon photodiode, or photomultiplier tubes arranged either singly or in series for cascade amplification of the signal. Photon counting electronics can be used for sensitive detection. The choice of detector will largely depend on the sensitivity and the excitation wavelength of detection required to carry out a particular assay. Note that the methods of the present teaching may involve either obtaining a full Raman or SERS spectrum across a range of wavelengths, or selecting a peak or peaks and scanning only at the wavelength of that peak(s) (i.e. Raman "imaging"). It is also possible to detect all Raman scattering using only a filter to remove reflected light, Raleigh scattering etc and a detector such as photodiode.

Apparatus for obtaining and/or analysing a Raman or SERS spectrum will almost certainly include some form of data processor such as a computer. Raman signals consist of a series of discrete spectral lines of varying intensity. The frequencies and the relative intensities of the lines are specific to the dye label being detected and the Raman/SERS signal is therefore a "fingerprint" of the label. If a Raman/SERS analyser is being used selectively to detect a mixture of *Listeria* spp. then it may be necessary to detect the "fingerprint" spectrum for multiple labels/dyes. Once the Raman/ SERS signal has been captured by an appropriate detector, its frequency and intensity data will typically be passed to a computer for analysis. Either the fingerprint Raman spectrum will be compared to reference spectra for identification of the detected Raman active compound or the signal intensity at the measured frequencies will be used, or multivariate analysis may be employed, to calculate the amount of Raman active compound detected.

A commercial Raman/SERS analyser of use in carrying out the invention would be expected to comprise the following components : a laser light source, the appropriate optics for carrying the light to the SERS active surface, an optional stage for mounting the sample for analysis, optics for receiving the Raman signal, a detector for converting the Raman signal into a series of intensities at certain wavelengths and a data processor for interpreting the wavelength/intensity data and/or multiple signal differentiation and providing an analytical output. The analyser may also comprise a database of wavelength signals representative of particular analytes in order to be able to detect and indicate to the user the analyte detected, as well as the analyte concentration, based upon the database of representative signals.

When provided in the form of particles, the first and/or second particles may be provided in a dry form, which are to be reconstituted by a liquid, such as a liquid comprising the sample to be investigated. For example, a sample may be obtained from an industrial process, such as a food production process and mixed with a liquid, such as an aqueous liquid, which in turn is used to reconstitute the first and/or second particles. A surface may also be investigated for bacterial contamination. For example, a swab may be taken of a surface and the swab contacted with a liquid, such as an aqueous liquid, in order that any bacteria, which may be present on the surface, are released into the liquid. In turn the liquid can be used to reconstitute the first and/or second particles. In an alternative example, a sample may be reconstituted from whole food and optionally mixed with a liquid, which in turn may be used to reconstitute the first and/or second particles.

The first and second antibodies, or fragments thereof, may be permitted to come into contact for a period of time, such as 30 seconds, 1, 2, 5 or 10 minutes, for example, in order to allow any detectable complexes to be formed. However, in some instances, no incubation period may be required.

Thus, in a second aspect, there is provided a sandwich immunoassay method for use in detecting and optionally quantifying *Listeria* in a sample, the immunoassay method comprising:
a) providing
   i) a first *Listeria* specific antibody, or antigen binding fragment thereof, immobilised to a first surface, wherein the first surface comprises the surface of a magnetic particle, paramagnetic particle, or nanoparticle;
   ii) a second *Listeria* specific antibody, or antigen binding fragment thereof, immobilised to a surface of a particle or bead, wherein the particle or bead further comprises a SERS or Raman label or dye; and
   iii) a liquid comprising a sample to be analysed;
   wherein the first and second *Listeria* specific antibodies, or antigen binding fragments thereof, are capable of binding to distinct antigens on the surface of a *Listeria* bacterium, in order to form a detectable complex comprising a *Listeria* bacterium sandwiched between said first and second *Listeria* specific antibodies, and wherein the first *Listeria* specific antibody, or antigen binding fragment thereof, is capable of binding any *Listeria* species and the second *Listeria* specific antibody, or antigen binding fragment thereof, is capable of binding a specific *Listeria* species;
b) permitting the first and second *Listeria* specific antibodies, or antigen binding fragments thereof and the liquid to come into contact for a period of time, in order that any detectable complexes may form between the first *Listeria* specific antibody, or antigen binding fragment thereof, the second *Listeria* specific antibody, or antigen binding fragment thereof and any *Listeria* present in the liquid; and
c) detecting any complexes which are formed, in order to detect and optionally identify any *Listeria* in the sample.

Any of the teachings in relation to the first aspect above, may be applied to the second aspect, unless otherwise described.

Contacting may include a mixing step, in order to permit the first and second *Listeria* specific antibodies, or antigen binding fragments thereof and any antigen to come into contact. Unlike prior art assays, the inventors have observed that a relatively short mixing time (such as less than 1min, 30 sec, 15 sec, 10 sec, or 5 sec) is sufficient, speeding up assay time. Moreover, a very short (e.g. less than 5 min, 3 min, 2 min, 1 min, 30 sec, or no further) incubation time may be required before commencing detection.

When the first and second *Listeria* specific antibodies or antigen binding fragments thereof are immobilised on the surface of a particle or bead, any detectable complexes, which are formed, will initially be in solution. As such, any detectable complexes may be, at least initially, retained magnetically using magnetic means. The magnetic means may comprise one or more permanent or electromagnets for generating magnetic field gradients within the liquid. In one embodiment, the magnet is a permanent magnet, such as a permanent magnet of a rare earth alloy such as anisotropic sintered materials composed of neodymium-iron-boron or samarium-cobalt. The magnet may be disposed external to a well, container, channel containing the liquid. The magnetic field acts to confine or aggregate the magnetic particles. A carefully balanced magnetic field strength in the liquid will pull the particles out of suspension into an aggregate, but will not be so strong as to overly compress the aggregate. Accordingly, a desired magnetic field strength within the magnetic field of the liquid may be created by appropriately adjusting the distance between the magnet and the liquid. There may be means provided for adjusting the distance between a magnet and the liquid.

In one teaching, where the first *Listeria* specific antibodies, or antigen binding fragments thereof, are immobilised to the surface of magnetic or paramagnetic particles, any detectable complexes are aggregated using a magnetic force, as described above, prior to detecting any complexes which are formed. Optionally, a wash step may be provided, prior to detection, in order to remove any non-magnetically bound material. Evidence provided herein, shows that improved detection may be observed if any detectable complexes are permitted to be in solution rather than magnetically accumulated on a surface. Thus, in one teaching, after applying a magnetic force, in order to accumulate any detectable complexes and following an optional wash step, the magnetic force is reduced or removed, so that any detectable complexes are permitted to return into solution.

A permanent or electromagnetic force may be reduced or increased, such as by moving a permanent magnet closer to, or further away from the liquid to be interrogated, or by increasing or decreasing the intensity of the applied field. Accumulation of any complexes using a permanent or electromagnetic force may be carried out for a period of time, before detection takes place. Typically, this may be for less than 10min, 5min, 3min, 2min, or 1min, for example.

Detection may be carried out using suitable detection means for detecting any detectable complexes formed. The detection means may be any suitable means depending on the particular assay. For example, the detection means may be such that detectable complexes are detected using Raman spectroscopy, such as by using a hand-held Raman spectrometer, as described in more detail above.

The number of detectable complexes formed will be proportional to the concentration of *Listeria* in the liquid sample. The higher the *Listeria* concentration in the liquid, the more detectable complexes formed during the magnetic accumulation.

In one teaching, any of the product or method as disclosed herein may be used to detect one or more additional bacterial species. In one embodiment, the bacterial species for detection may be one or more other (non-*Listeria*) bacterial species, in addition to one or more *Listeria* species. In one embodiment, the detection of the present disclosure may comprise other bacterial species, such as *E. coli, Staphylococcus aureus,* methicillin-resistant *Staphylococcus aureus* and *Pseudomonas* species, for example. The skilled person would recognise that the product or method of the present disclosure may be applied for the detection of other bacterial species, such as by swapping the Listeria-specific antibody, or the Raman reporter molecule, with one or more antibodies that specifically target another bacterial species of interest, which are commercially available or known in the art.

The present disclosure will now be further described in detail and with reference to the Figures which show:

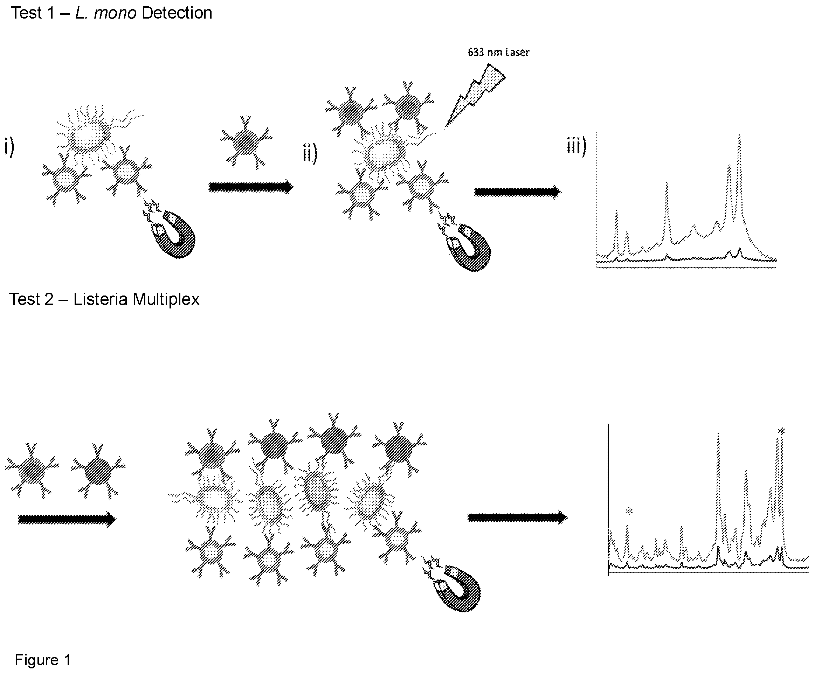

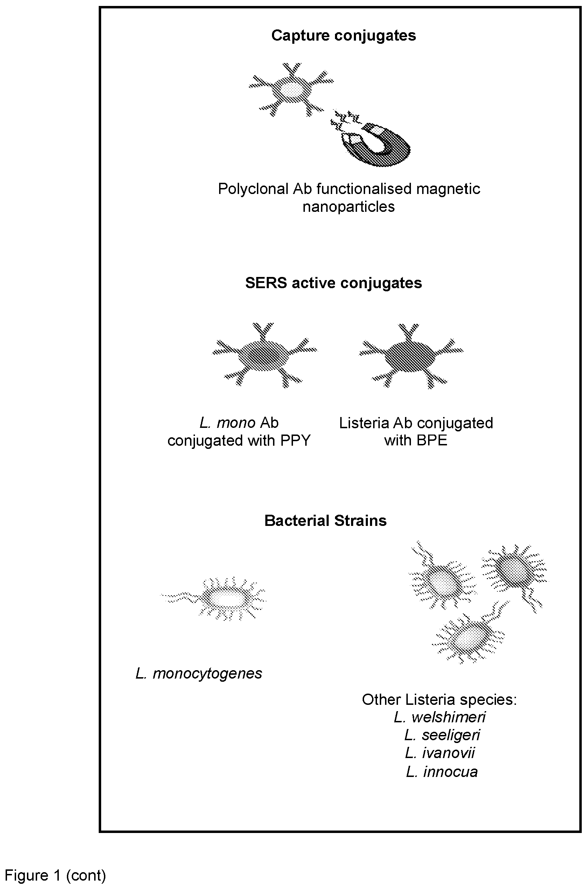

**Fig. 1****.** Schematic illustrating the single-plex and multiplex detection assay;

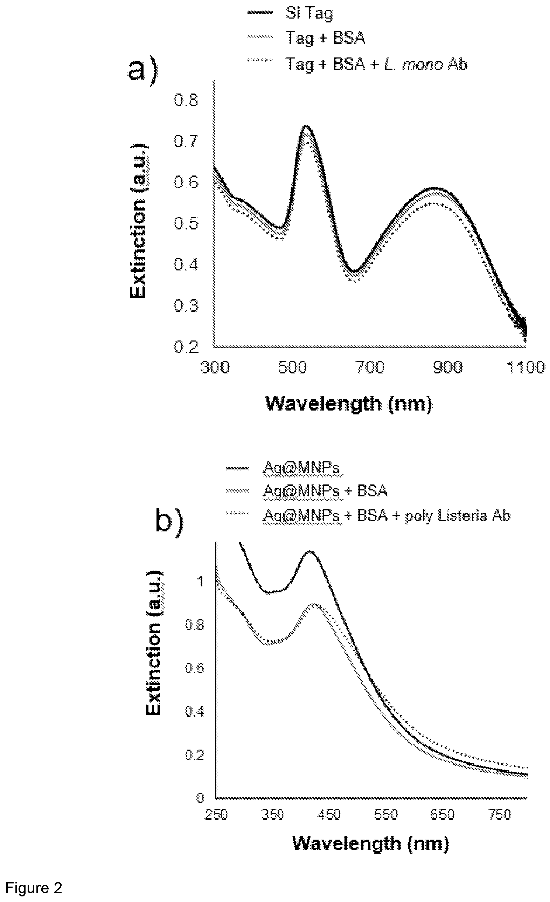

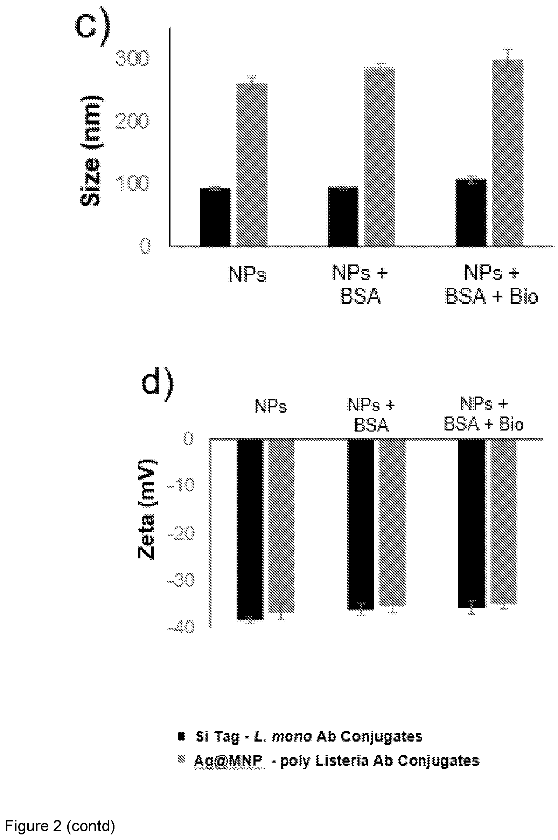

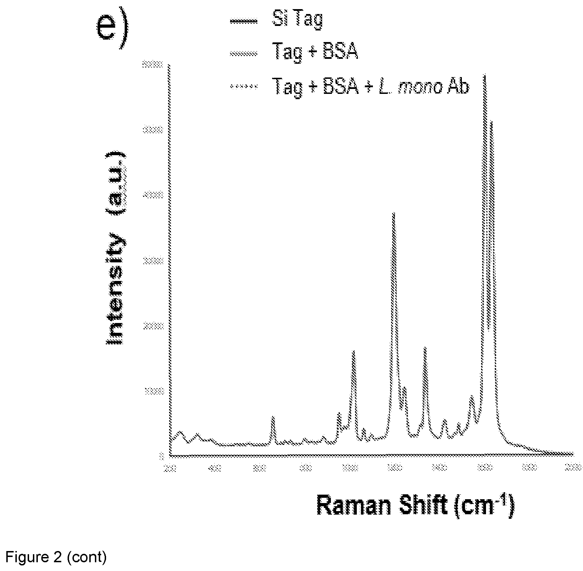

**Fig. 2****.** Characterisation of antibody-nanoparticle conjugates. a) Extinction spectra showing the conjugation steps involved in the preparation of SERS active *L. mono* antibody functionalised Si tags. b) Extinction spectra showing the conjugation steps involved in the preparation of polyclonal *Listeria* antibody functionalised Ag@MNPs. Note, a passive adsorption approach was used for conjugating the antibodies to nanoparticles with BSA used as a blocking agent. c) dynamic light scattering and d) zeta potential for both conjugates at each stage of the conjugation. e) SERS spectra obtained with a 638 nm SnRI spectrometer and acquisition time of 1 s; showing the change in spectrum at each step of the conjugation process for *L. mono* antibody functionalised Si tags only. The mean of 3 replicate samples is shown along with standard deviation error bars;

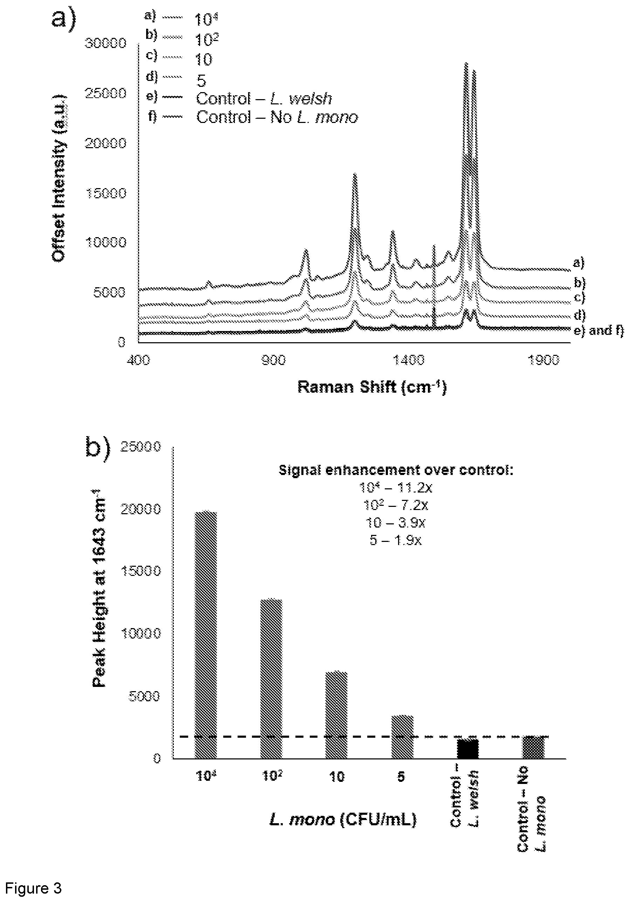

**Fig. 3****.** Assay was performed for the detection of *L. mono* over the bacteria concentration range 10⁴ to 5 CFU/mL. a) Comparison of SERS spectra showing changes in BPE signal and b) the associated bar charts showing the SERS peak intensities at each of the bacteria concentrations, and these are compared to control samples (when no bacteria are present; - and when a non-specific species *(L. welsh)* is added)). The characteristic peak at 1643 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls;

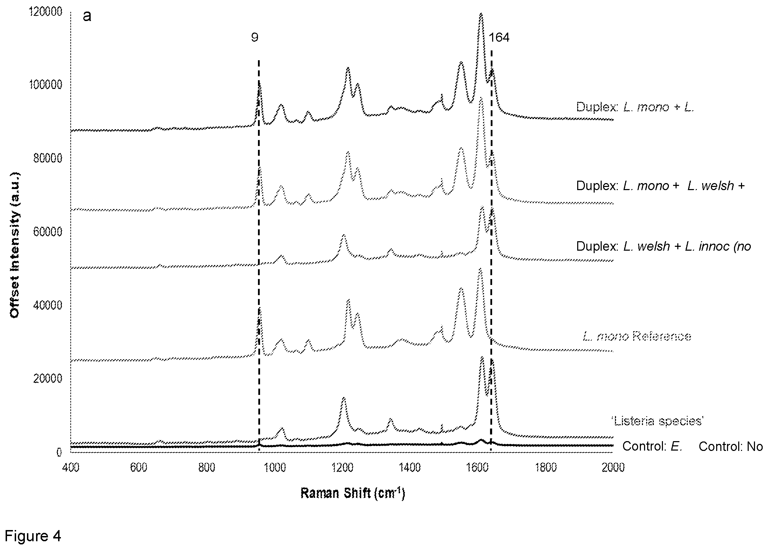

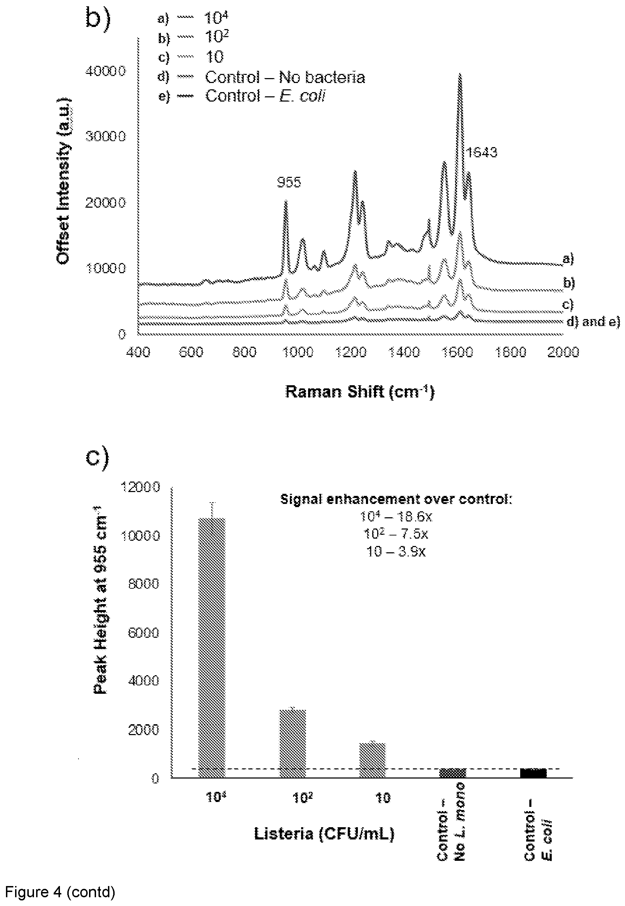

**Fig. 4****.** SERS spectra showing when multiple *Listeria* species are present. (a) Stacked SERS spectra showing the spectra obtained when multiple *Listeria* species were present; when *Listeria* species were present but *L. mono* was absent; control samples for comparing when no bacteria was added or when non-specific bacteria (*E*. *coli)* was added and also the SERS spectra obtained from the detection of *L. mono* and *L. welsh* individually. The black dotted lines show peaks that are unique to each Raman reporter and hence used to identify the presence of the bacterial targets. The bacteria concentration used was 10⁴ CFU/mL. (b) *Listeria* multiplex assay was performed over the bacteria concentration range 10⁴ to 10 CFU/mL, with a 1:1 concentration ratio for *L. mono* to *L. welsh.* The SERS spectra compares the changes in PPY signal and c) the associated bar charts showing the SERS peak intensities at each of the bacteria concentrations, and these are compared to control samples (when no bacteria are present; and when a non-specific species (*E.coli*) is added). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line in Figure 4c gives visual clarification of the SERS peak intensity of the controls;

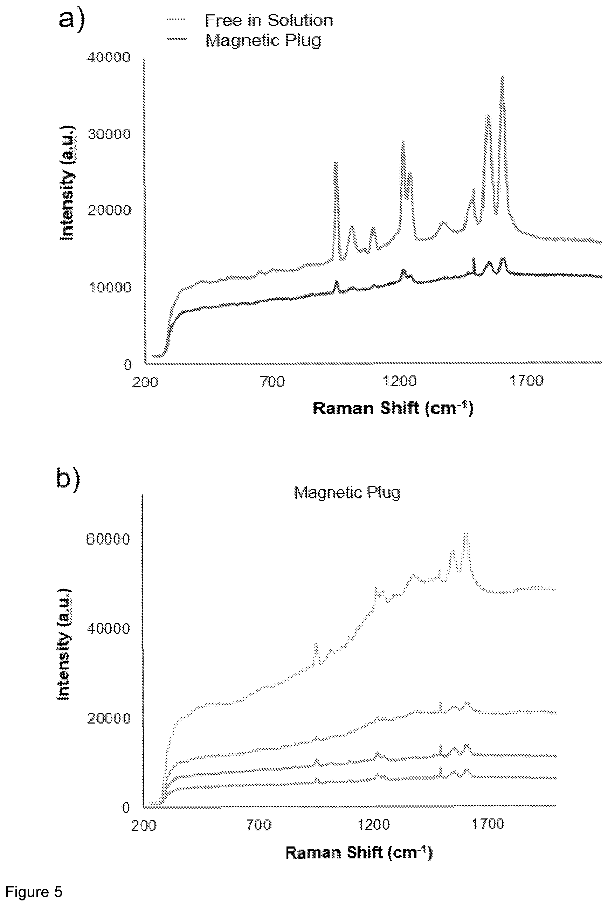

**Fig. 5****.** SERS spectra for the detection of *L. mono* at a concentration of 10⁴ CFU/mL a) Comparison of SERS spectra, when bacterial-complex is interrogated whilst captured on magnetic plug versus when it's free in solution. b) SERS spectra showing the variability when using the magnetic plug for detection. SERS spectra were recorded using a 633 nm laser excitation with an accumulation time of 1 s. The sample was collected on the magnet for 25 mins before interrogating with laser;

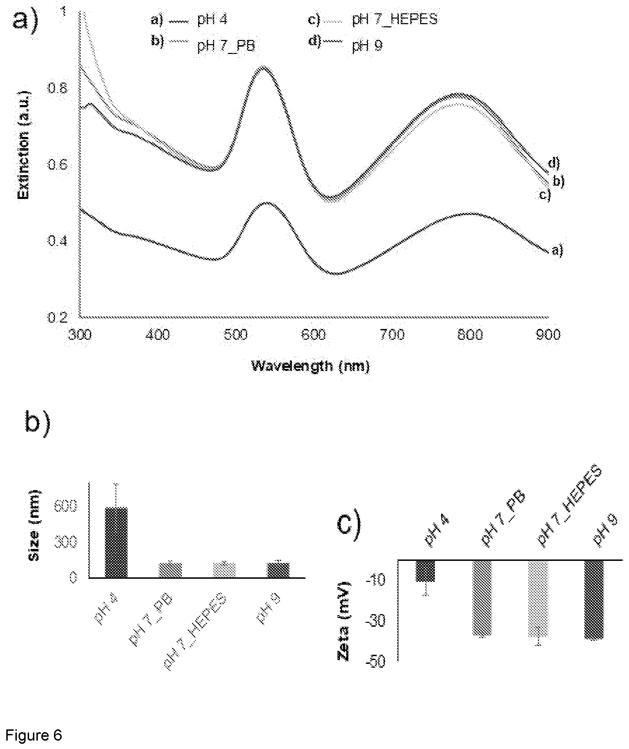

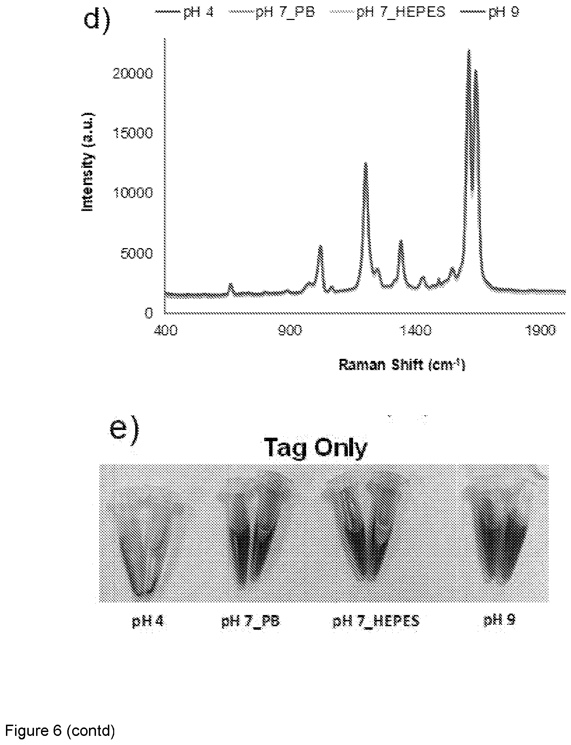

**Fig. 6****.** Characterisation of Si tags across pH range 4 to 9 a) Comparison of extinction spectra at differing pH's b) DLS, c) zeta potential d) SERS spectra obtained with a 633 nm Wasatch spectrometer and acquisition time of 0.1 s and e) picture of the Si tags after 3 hours. The mean of 3 replicate samples is shown along with standard deviation error bars;

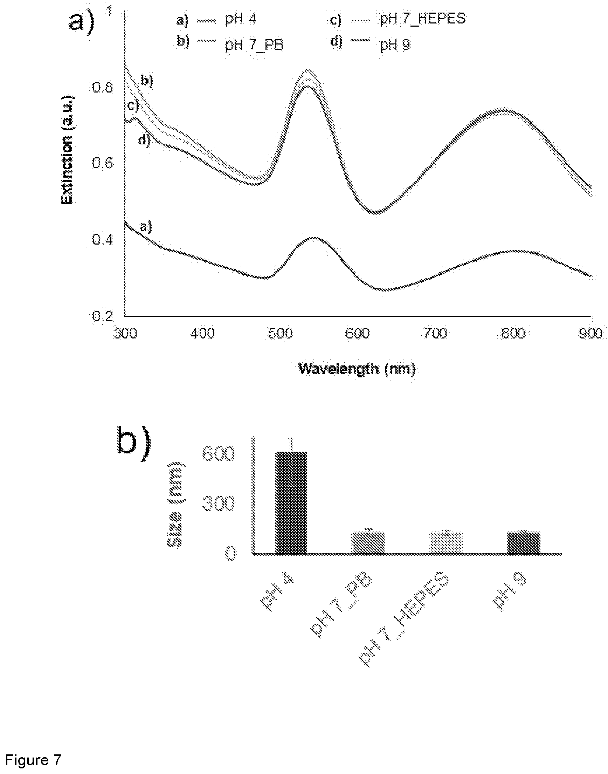

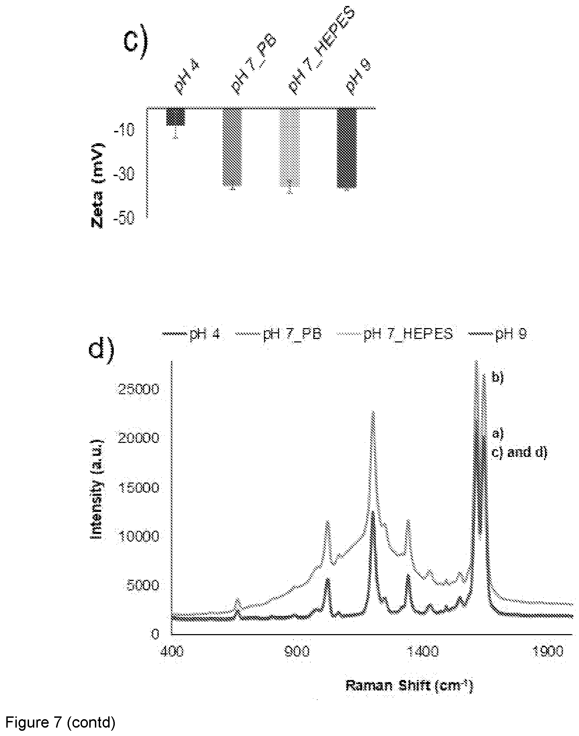

**Fig. 7****.** Characterisation of Tags + BSA across pH range 4 to 9 a) Comparison of extinction spectra at differing pH's b) DLS, c) zeta potential and d) SERS spectra obtained with a 633 nm Wasatch spectrometer and acquisition time of 0.1 s. The mean of 3 replicate samples is shown along with standard deviation error bars;

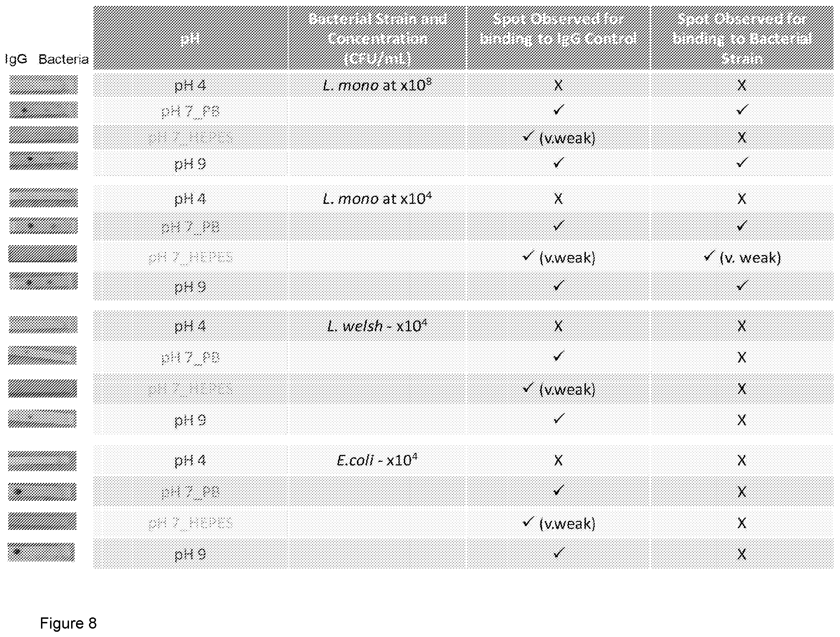

**Fig. 8****.** Lateral flow analysis for the Tag + *L. mono* Ab samples across pH range 4 to 9. Bacterial strains and the concentrations used were as follows: *L. mono* at 10⁸ and 10⁴ CFU/mL, and non-specific strains *L. welsh* and *E. coli* at 10⁴ CFU/mL. Anti-mouse IgG (0.5 mg/mL) was used as a control to confirm if antibodies are present on the Si tag surface; and

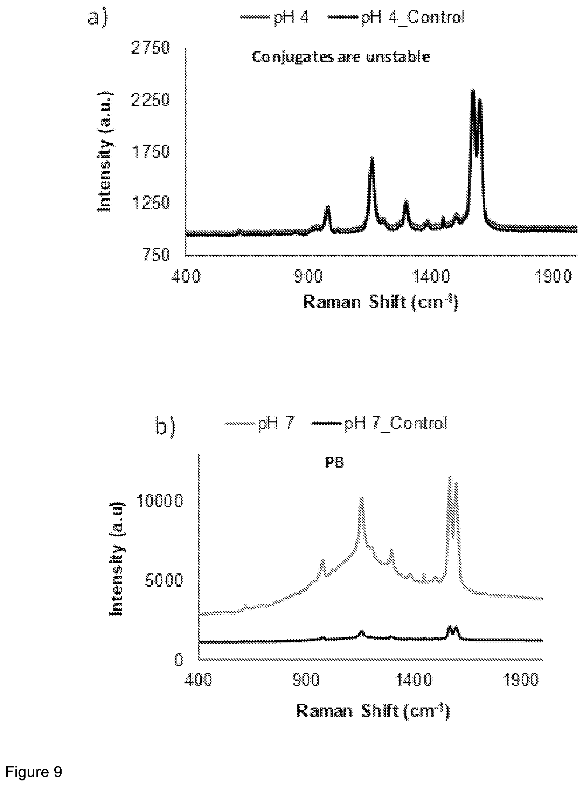

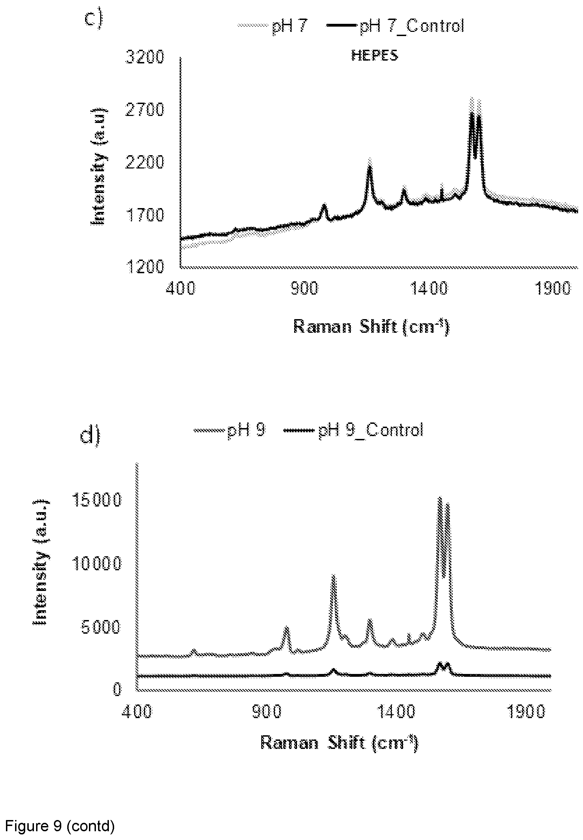

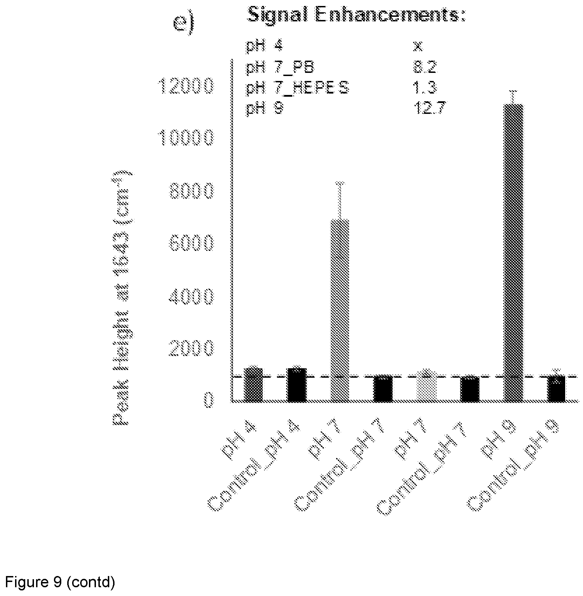

**Fig. 9****.** Assay was performed for the detection of *L. mono* at different pH's with a bacterial concentration of 10⁴ CFU/mL. Comparison of SERS spectra at a) pH 4 in acetate buffer, b) pH 7 in PB buffer, c) pH 7 in HEPES buffer, d) pH 9 in borate buffer and e) the associated bar charts showing the SERS peak intensities at each of the pH's, and these are compared to control samples (when no bacteria are present). The characteristic peak at 1643 cm⁻¹ was used to calculate the peak intensities. SERS spectra were recorded using a 633 nm laser excitation with an accumulation time of 0.1 s. Peak intensities were obtained by scanning 3 replicate samples 5 times, and the error bars represent one standard deviation. The dashed line in gives visual clarification of the SERS peak intensity of the controls.

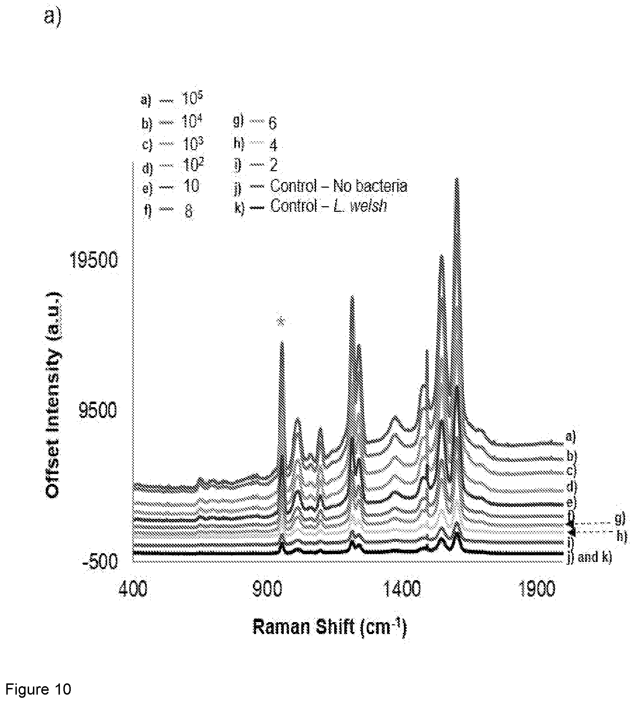

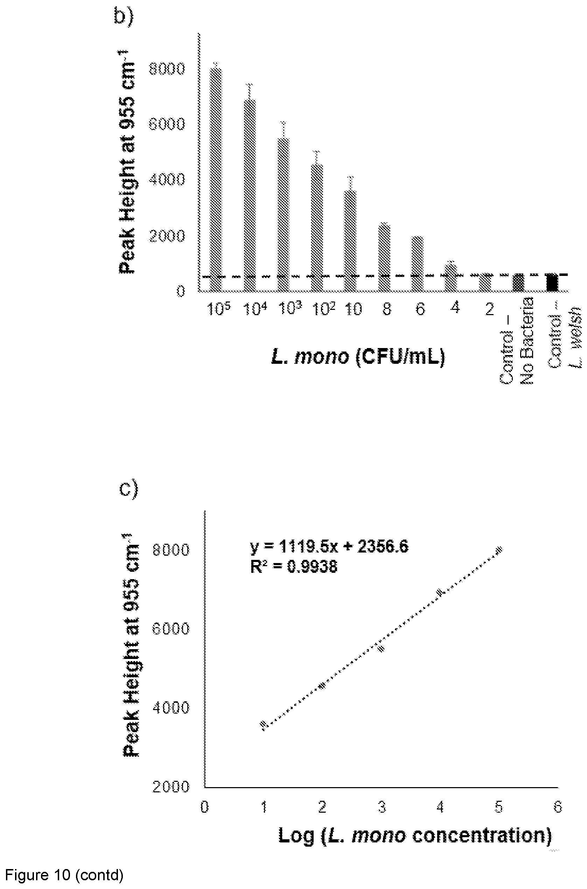

**Fig. 10** Assay was performed for the detection of *L. mono* over the bacteria concentration range 10⁵ to 2 CFU/mL. a) Comparison of SERS spectra showing changes in PPY signal and b) the associated bar charts showing the SERS peak intensities at each of the bacteria concentrations, and these are compared to control samples (when no bacteria are present; and when non-specific species *(L. welsh)* is added). The characteristic peak at 955 cm⁻¹ was used to calculate the peak intensities. c) Concentration study over the bacterial range 10⁵ to 10 CFU/mL which is equivalent to 5 to 1 on log concentration scale. The equation of the line allows for limits of detection to be determined and unknown samples to be quantified. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

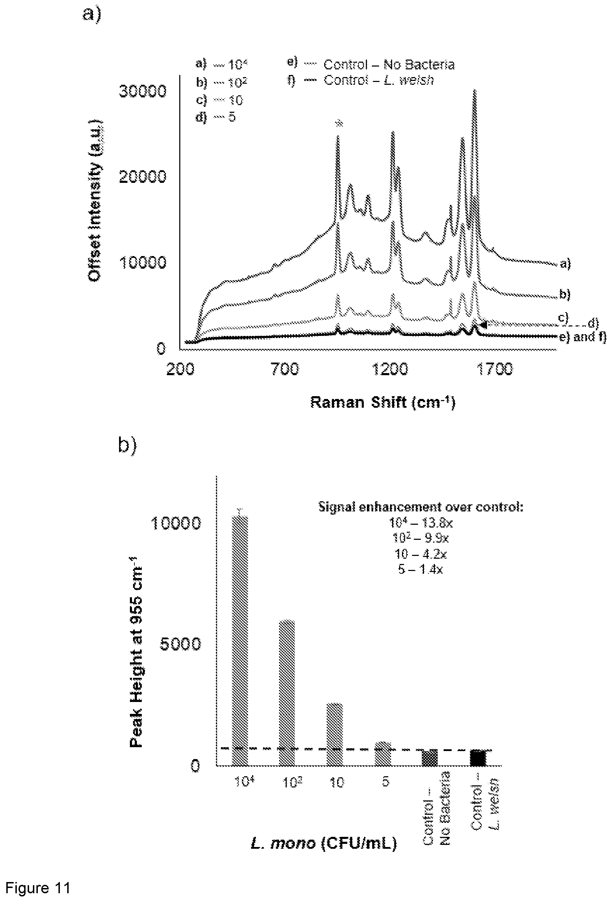

**Fig. 11** Concentration study of *L. mono* spiked in a Food Matrix over the bacteria concentration range 10⁴ to 5 CFU/mL. The food matrix consisted of a blended chicken and bacon sandwich in MRD. a) Comparison of SERS spectra showing changes in PPY signal and b) the associated bar charts showing the SERS peak intensities at each of the bacteria concentrations, and these are compared to control samples (when no bacteria are present; and when non-specific species *(L. welsh)* is added). The characteristic peak at 955 cm⁻¹ was used to calculate the peak intensities. SERS spectra were recorded using a 633 nm laser excitation with an accumulation time of 0.1 s. Peak intensities were obtained by scanning 3 replicate samples 5 times, and the error bars represent one standard deviation. The dashed line gives visual clarification of the SERS peak intensity of the controls.

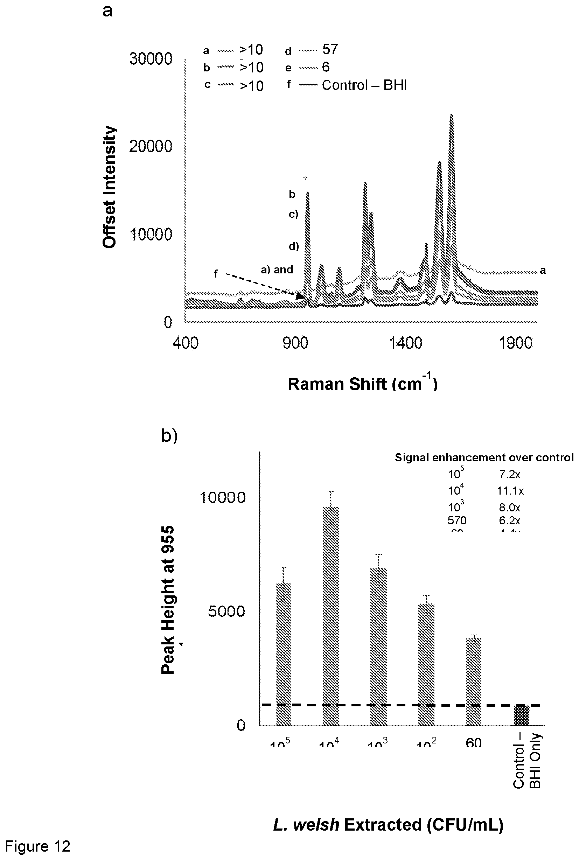

**Fig. 12** Swab extraction of Listeria from a surface and SERS analysis performed using the magnetic assay. Assay was performed for the detection of *L. welsh* (non-pathogenic strain) over the bacteria concentration range 10⁵ to 60 CFU/mL. a) Comparison of SERS spectra showing changes in PPY signal and b) the associated bar charts showing the SERS peak intensities at each of the bacteria concentrations, and these are compared to a control sample (when no bacteria are present; BHI broth only). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

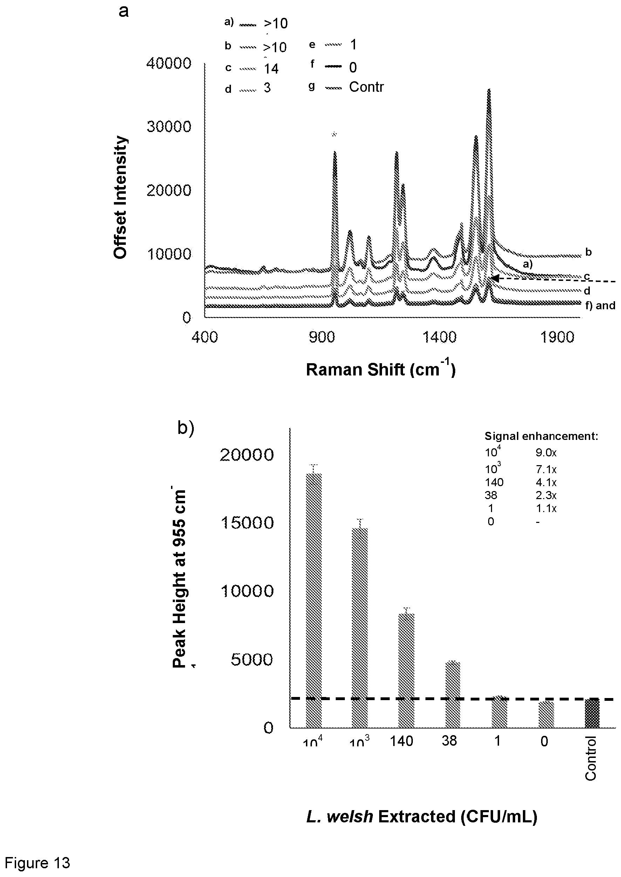

**Fig. 13** Swab extraction of Listeria spiked in a food matrix from a surface and then SERS analysis performed using the magnetic assay. The food matrix consisted of a blended chicken and bacon sandwich in MRD. Assay was performed for the detection of *L. welsh* (nonpathogenic strain) over the bacteria concentration range 10⁴ to 0 CFU/mL. a) Comparison of SERS spectra showing changes in PPY signal and b) the associated bar charts showing the SERS peak intensities at each of the bacteria concentrations, and these are compared to a control sample (when no bacteria are present; BHI broth + FM). The characteristic peak at 955 cm⁻¹ was used to calculate the peak intensities. SERS spectra were recorded using a 633 nm laser excitation with an accumulation time of 0.1 s. Peak intensities were obtained by scanning 3 replicate samples 5 times, and the error bars represent one standard deviation. The dashed line gives visual clarification of the SERS peak intensity of the controls.

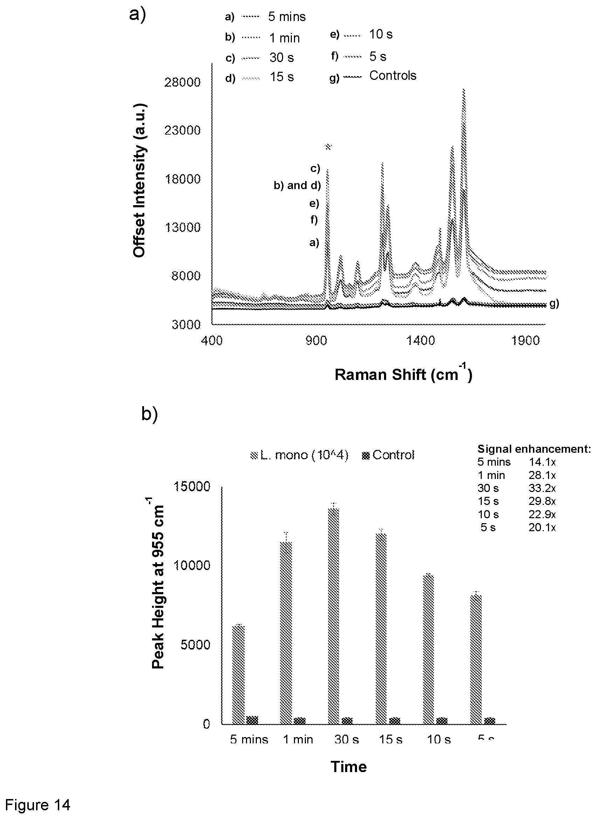

**Fig. 14** Time study to determine how long it takes for the biological interaction to occur between the bacteria and the Ab conjugates using a vortex with vigorous shaking (speed at 3000 rpm). *L. mono* at a concentration of 10⁴ CFU/mL was used throughout and the mixing time was reduced from 5 mins to 5 secs. a) Comparison of SERS spectra showing changes in PPY signal and b) the associated bar charts showing the SERS peak intensities at each of the bacteria concentrations, and these are compared to control samples at each of the time points (when no bacteria are present). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation.

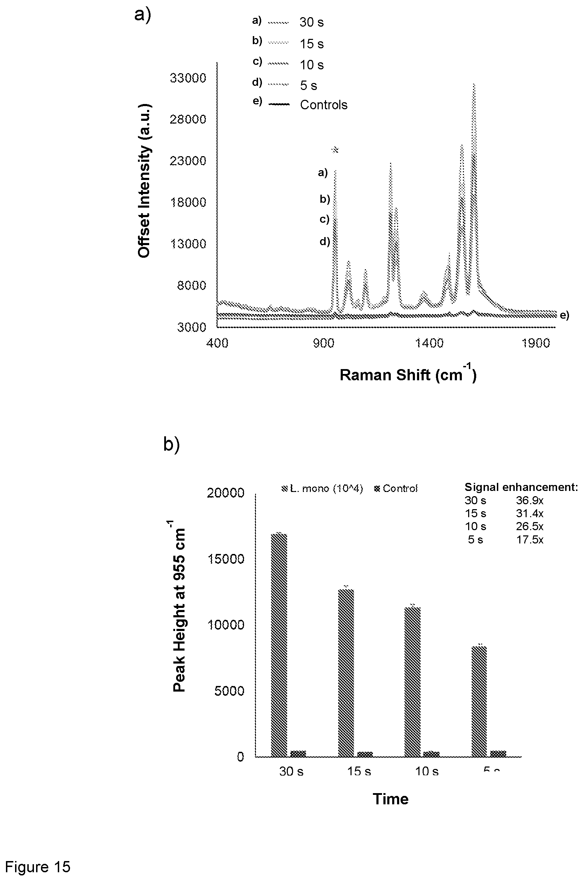

**Fig. 15** Time study to determine how long it takes for the biological interaction to occur between the bacteria and the Ab conjugates when mixing is performed by hand i.e. shaking the Eppendorf. *L. mono* at a concentration of 10⁴ CFU/mL was used throughout and the mixing time was reduced from 30 secs to 5 secs. a) Comparison of SERS spectra showing changes in PPY signal and b) the associated bar charts showing the SERS peak intensities at each of the bacteria concentrations, and these are compared to control samples at each of the time points (when no bacteria are present). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation.

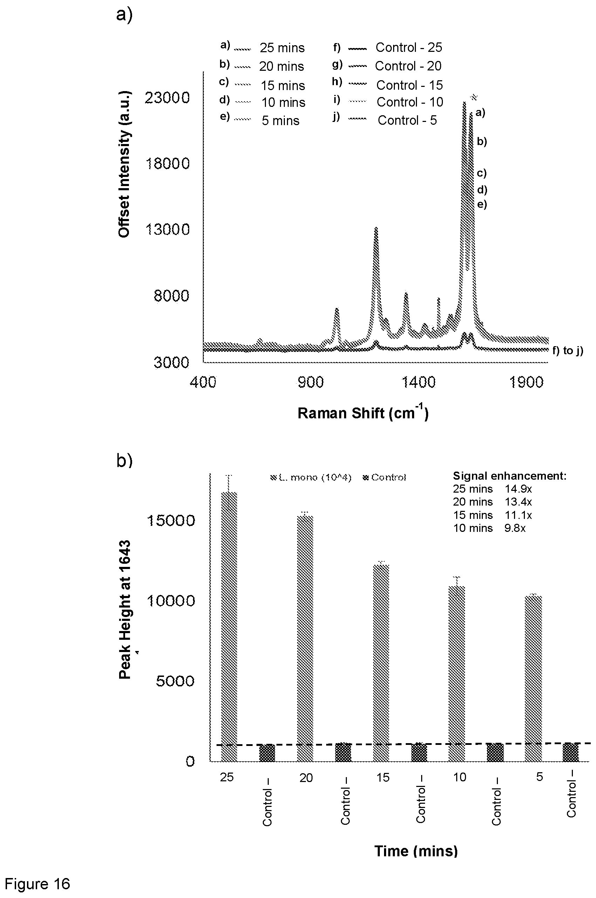

**Fig. 16** Time study to determine how long it takes to capture the 'bacterial complex' without impacting the signal enhancements (discrimination between sample and control). The mixing time remained at 5 mins, but the magnetic separation time was reduced from 25 to 5 mins. *L*. *mono* at a concentration of 10⁴ CFU/mL was used throughout. a) Comparison of SERS spectra showing changes in BPE signal and b) the associated bar charts showing the SERS peak intensities at each of the bacteria concentrations, and these are compared to control samples at each of the time points (when no bacteria are present). The characteristic peak at 1643 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

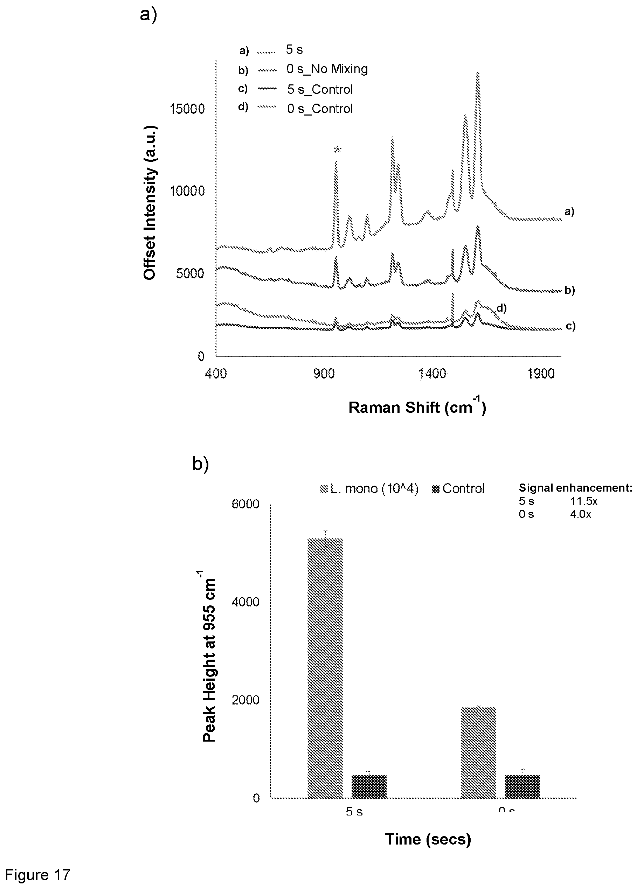

**Fig. 17** Time study to determine if the assay can be performed with both, a reduced magnetic separation time and mixing time. *L. mono* at a concentration of 10⁴ CFU/mL was used throughout. The magnetic separation time was 5 mins with a mixing time of 5s and 0s (no mixing). a) Comparison of SERS spectra showing changes in PPY signal and b) the associated bar charts showing the SERS peak intensities at each of the bacteria concentrations, and these are compared to control samples at each of the time points (when no bacteria are present). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation.

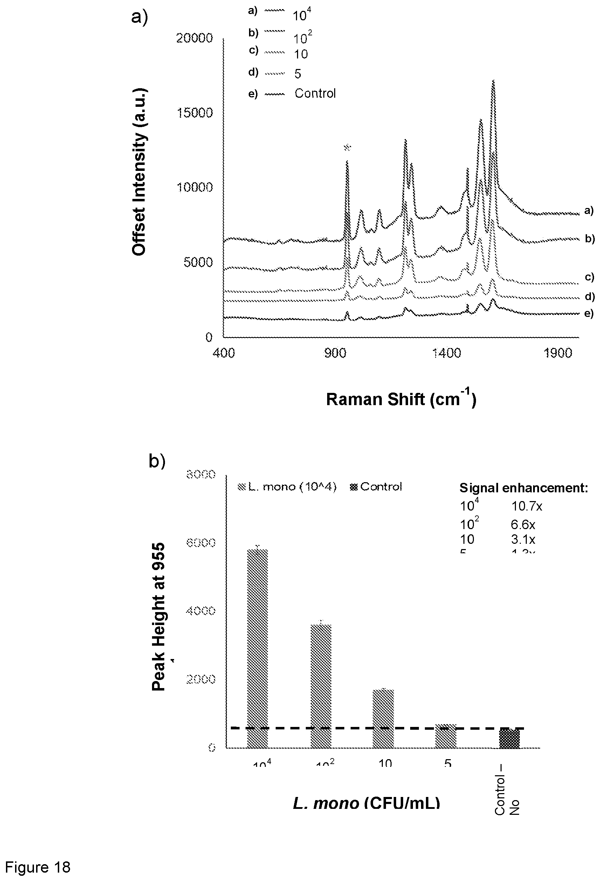

**Fig. 18** Assay was performed for the detection of *L. mono* over the bacteria concentration range 10⁴ to 5 CFU/mL using the conditions that allow detection in 6 mins i.e. 5 sec mixing plus 5 mins magnetic separation a) Comparison of SERS spectra showing changes in PPY signal and b) the associated bar charts showing the SERS peak intensities at each of the bacteria concentrations, and these are compared to a control sample (when no bacteria are present). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

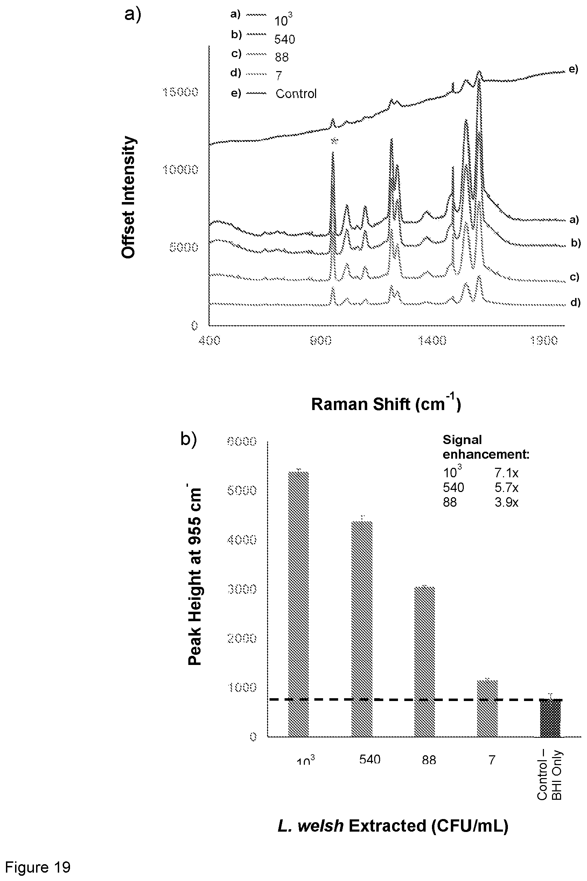

**Fig. 19** Swab extraction of Listeria from a surface and SERS analysis performed using the assay with the conditions that allow detection in 6 mins i.e. 5 sec mixing plus 5 mins magnetic separation. Assay was performed for the detection of *L. welsh* (non-pathogenic strain) over the bacteria concentration range 10³ to 7 CFU/mL. a) Comparison of SERS spectra showing changes in PPY signal and b) the associated bar charts showing the SERS peak intensities at each of the bacteria concentrations, and these are compared to a control sample (when no bacteria are present; BHI broth only). The characteristic peak at 955 cm⁻¹ was used to calculate the peak intensities. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

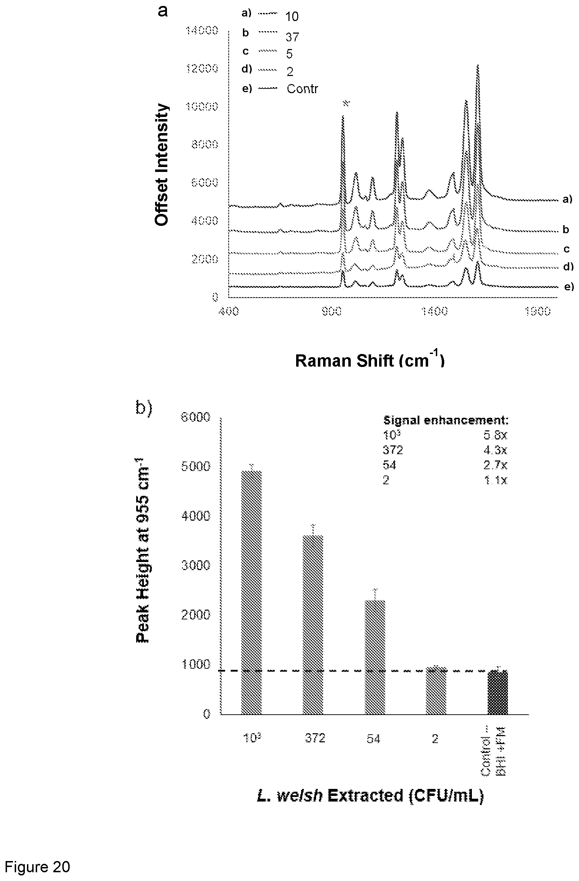

**Fig. 20** Swab extraction of Listeria spiked in a food matrix from a surface and then SERS analysis performed using the assay with the conditions that allow detection in 6 mins i.e. 5 sec mixing plus 5 mins magnetic separation. The food matrix consisted of a blended cheese and ham sandwich in MRD. Assay was performed for the detection of *L. welsh* (non-pathogenic strain) over the bacteria concentration range 10³ to 2 CFU/mL. a) Comparison of SERS spectra showing changes in PPY signal and b) the associated bar charts showing the SERS peak intensities at each of the bacteria concentrations, and these are compared to a control sample (when no bacteria are present; BHI broth + FM). The characteristic peak at 955 cm⁻¹ was used to calculate the peak intensities. SERS spectra were recorded using a 633 nm laser excitation with an accumulation time of 0.1 s. Peak intensities were obtained by scanning 3 replicate samples 5 times, and the error bars represent one standard deviation. The dashed line gives visual clarification of the SERS peak intensity of the controls.

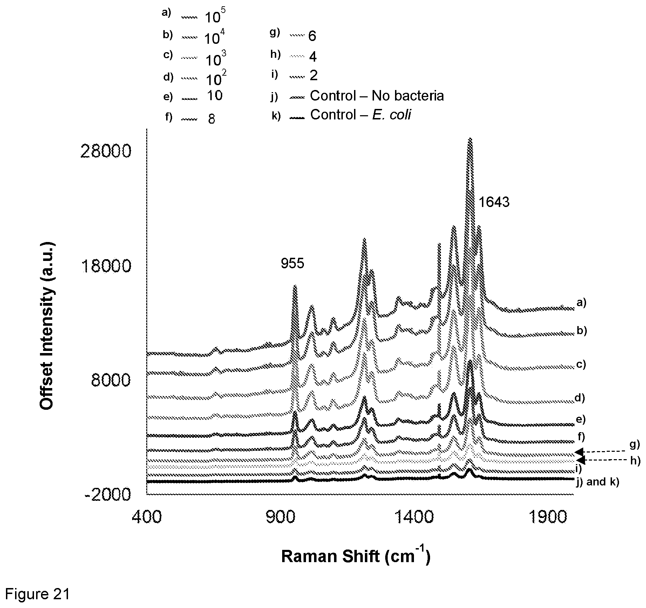

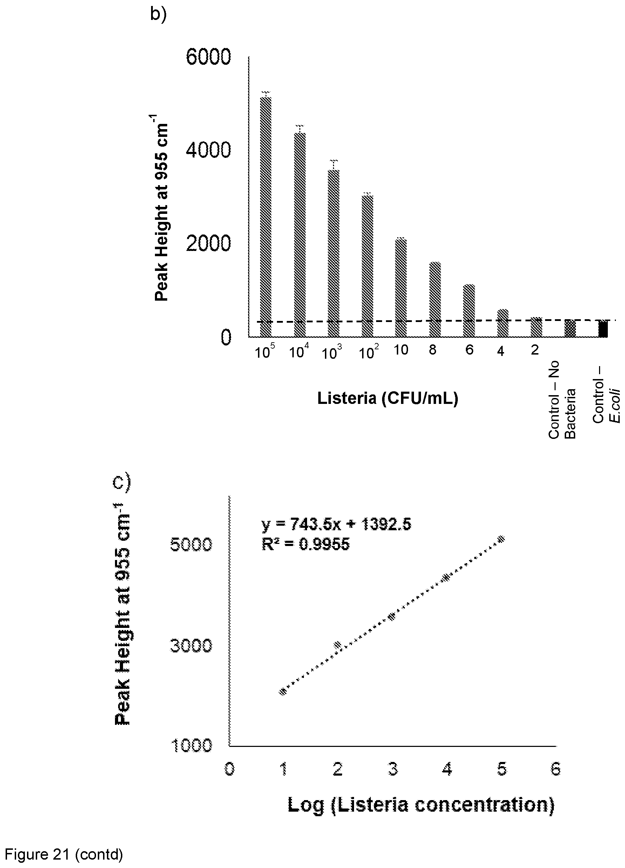

**Fig. 21** Duplex assay was performed for the detection of Listeria over the bacteria concentration range 10⁵ to 2 CFU/mL; with a 1:1 concentration ratio for *L. mono* to *L. welsh.* a) Comparison of SERS spectra showing changes in PPY signal and b) the associated bar charts showing the SERS peak intensities at each of the bacteria concentrations, and these are compared to control samples (when no bacteria are present; and when non-specific species (*E. coli*) is added)). The characteristic peak at 955 cm⁻¹ was used to calculate the peak intensities. c) Concentration study over the bacterial range 10⁵ to 10 CFU/mL which is equivalent to 5 to 1 on log concentration scale. The equation of the line allows for limits of detection to be determined and unknown samples to be quantified. SERS spectra were recorded using a 633 nm laser excitation with an accumulation time of 1 s. Peak intensities were obtained by scanning 3 replicate samples 5 times, and the error bars represent one standard deviation. The dashed line gives visual clarification of the SERS peak intensity of the controls.

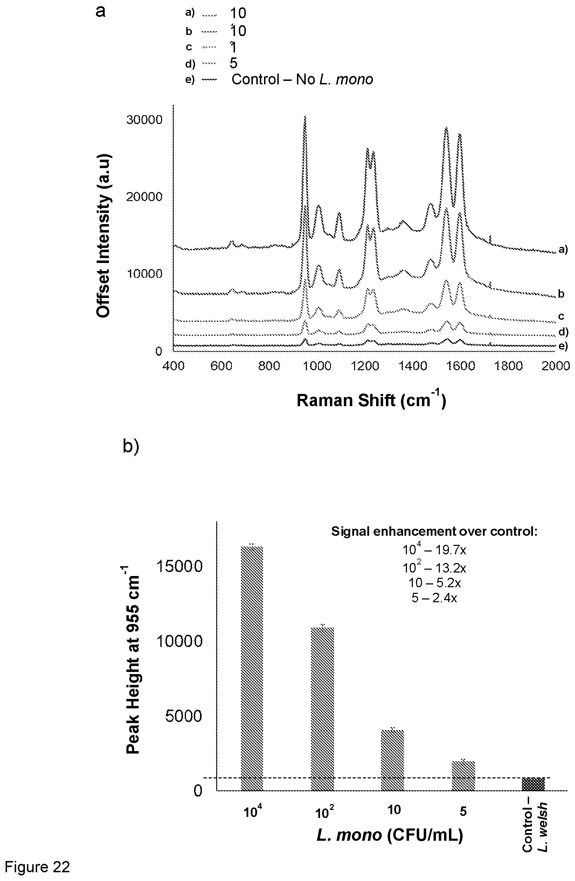

**Fig. 22** Assay was performed for the detection of *L. mono* over the bacteria concentration range 10⁴ to 5 CFU/mL. a) Comparison of SERS spectra showing changes in PPY signal and b) the associated bar charts showing the SERS peak intensities at each of the bacteria concentrations, and these are compared to control samples (when no bacteria are present and when non-specific species *(L. welsh)* is added). The characteristic peak at 955 cm⁻¹ was used to calculate the peak intensities. SERS spectra were recorded using a handheld CBEx Raman spectrometer with a 785 nm laser excitation and an accumulation time of 1 s. Peak intensities were obtained by scanning 3 replicate samples 5 times, and the error bars represent one standard deviation. The dashed line gives visual clarification of the SERS peak intensity of the controls.

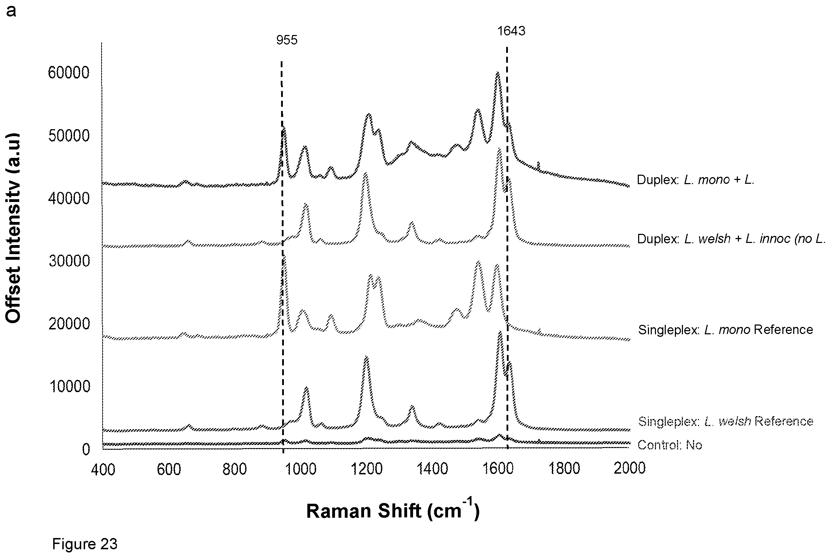

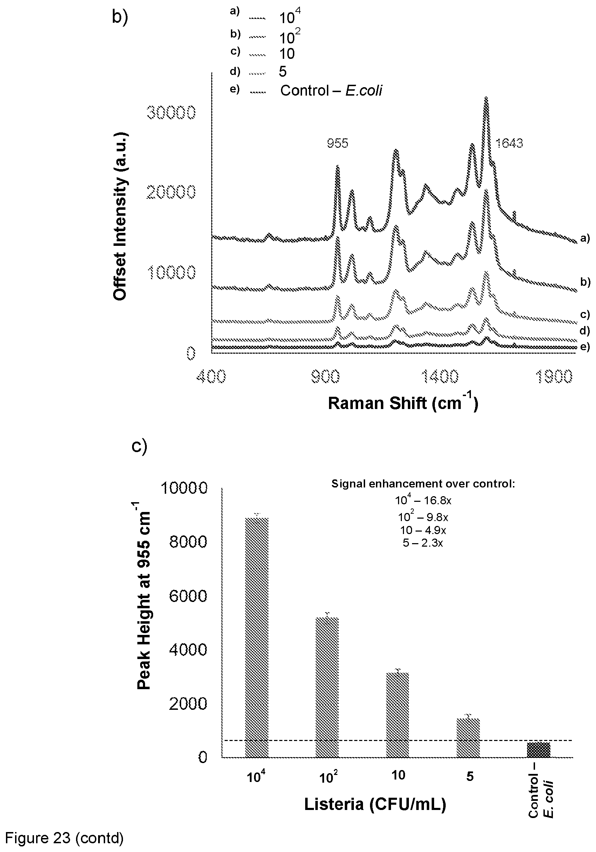

**Fig. 23** SERS spectra showing when multiple Listeria species are present. (a) Stacked SERS spectra showing the spectra obtained when multiple Listeria species were present (top); when Listeria species were present but *L. mono* was absent (second from top); control samples for comparing when no bacteria was added (bottom) or when non-specific bacteria (*E. coli -* bottom) was added and also the SERS spectra obtained from the detection of *L. mono* (third from top) and *L. welsh* (fourth from top) individually. The black dotted lines show peaks that are unique to each Raman reporter and hence used to identify the presence of the bacterial targets. The bacteria concentration used was 10⁴ CFU/mL. (b) Listeria multiplex assay was performed over the bacteria concentration range 10⁴ to 5 CFU/mL, with a 1:1 concentration ratio for *L. mono* to *L. welsh.* The SERS spectra compares the changes in PPY signal and c) the associated bar charts showing the SERS peak intensities at each of the bacteria concentrations, and these are compared to control samples (when no bacteria are present; and when non-specific species (*E. coli*) is added). The characteristic peak at 955 cm⁻¹ was used to calculate the peak intensities. SERS spectra were recorded using a handheld CBEx Raman spectrometer with a 785 nm laser excitation and an accumulation time of 1 s. Peak intensities were obtained by scanning 3 replicate samples 5 times, and the error bars represent one standard deviation. The dashed line in Figure 23c gives visual clarification of the SERS peak intensity of the controls.

### Materials and methods

**Chemicals and Antibodies:** sodium tetrachloroaurate (III) dihydrate, sodium citrate, 4-(1H-pyrazol-4-yl)pyridine (PPY), 1,2-bis(4-pyridyl)ethylene (BPE), methanol, (3-aminopropyl)triethoxysilane (APTES), sodium silicate, iron(II)chloride tetrahydrate, iron(lll)chloride hexahydrate, sodium hydroxide (anhydrous), glucose, calcium nitrate tetrahydrate, magnesium nitrate hydrate, sodium dihydrate phosphate monohydrate, sodium tetraborate, sodium acetate, phosphate buffer, N-(2-hydroxyethyl)piperazine-N'-(2-ethanesulfonic acid) (HEPES), acetic acid, boric acid, bovine serum albumin (BSA), hydrochloric acid, nitric acid, Tween 20, Luria-Bertani broth with agar (LB Miller), LB broth, brain heart infusion (BHI) agar and BHI broth were purchased from Sigma-Aldrich (Irvine, UK). 4-(1H-pyrazol4-yl)-pyridine (PPY) was purchased from Fluorochem (Hadfield, UK). Doubly distilled and deionised water (d.H₂O) was prepared in-house. The Raman reporters, BPE and PPY, were prepared in stock solutions by dissolving the solid in methanol. Subsequent dilutions were then carried out in d.H₂O.

*Listeria* antibodies (monoclonal and polyclonal) were purchased from Fitzgerald Industries International (Acton, USA) whilst the *Listeria* strains were purchased from LGC Standards Ltd.

### Synthesis of Nanoparticles

Synthesis of gold nanoparticles: Gold nanoparticles (AuNP) with an average size of ~50 nm were synthesised using a modified Turkevich method where citrate ions act as the reducing and capping agent for the NPs.¹ Sodium tetrachloroaurate (III) dihydrate (60.5 mg) was added to d.H₂O (500 mL) and heated to boiling. Sodium citrate (42 mg in 7.5 mL d.H₂O) was then added to the boiling solution and left for 15 minutes, before being left to cool. Vigorous stirring was maintained throughout.

Synthesis of SERS active silica coated gold nanotags: SERS active silica (Si) coated tags were synthesised using a method modified from Li et al.² Raman reporters (500 µL, 100 µM of BPE or PPY) were added separately to a solution of AuNPs (100 mL) under stirring. The solution was stirred until the SERS response was optimised or the solution became noticeably darker in colour (upto 5 minutes for BPE, 1 minute for PPY because the colour change occurred quickly, and this is indicative of aggregation). APTES (1.6 µL, undiluted) was then added, immediately followed by sodium silicate (111 µL, undiluted). The mixture was stirred vigorously at boiling for 30 minutes, and it was then removed from heat and stirred overnight. The Si tags were then centrifuged (7000g, 20 mins) and re-suspended in d.H₂O (30 mL).

Synthesis of magnetic nanoparticles: Maghemite (γ-Fe₂O₃) magnetic nanoparticles (MNPs) were prepared by co-precipitation, following a method previously reported by Kumar et al.³ Acidified iron salt solution was prepared by adding together iron(II)chloride tetrahydrate (1.98 g), iron(III)chloride hexahydrate (5.335 g) and concentrated hydrochloric acid (821 µL) and making the volume up to 25 mL with d.H₂O. Using a round-bottom flask, sodium hydroxide (15.1 g) was dissolved in d.H₂O (250 mL) and heated to 50 °C on a heating mantle. The acidified iron salt solution was then added dropwise with vigorous stirring and a black precipitate formed immediately. Stirring was continued for a further 20 min at 50 °C, then the solution left to settle and cool. The black precipitate was washed twice with d.H₂O (200 mL) and then nitric acid (125 mL; 0.1 M) was added and the solution heated to 95 °C with constant stirring, for 40 min. The resultant reddish-brown solution was centrifuged (3800g for 20 min) in triplicate and re-suspended in d.H₂O; this produced the stock maghemite MNPs.

Synthesis of silver coated magnetic nanoparticles: Ag@MNPs were prepared using a glucose reduction method, reported by Donnelly et al.⁴ Stock MNPs (1 mL) were added to a round bottom flask, along with glucose (0.25 g), silver nitrate (1%; 1.5 mL) and d.H₂O (4 mL). The mixture was sonicated for 10 min and then heated at 90 °C for 30 min while rotating. The resultant Ag@MNPs were centrifuged (3800g for 20 min) three times and re-dispersed in sodium citrate (5 mM; 6 mL). Note that magnetic collection of a solution of the synthesised Ag@MNPs resulted in a clear supernatant, indicating that no non-magnetic silver nanoparticles were formed during the silver nitrate reduction.

### Preparation of buffers:

*Acetate buffer (0.2 M):* sodium acetate (0.16 g) was dissolved in d.H₂O (10 mL). Sodium acetate solution (3.7 mL) was added to acetic acid (6.3 mL). The pH was then adjusted to pH 4.4.

*Borate buffer (100 mM):* sodium tetraborate (76 mg) was dissolved in d.H₂O (2 mL). Boric acid (15 mg) was also dissolved in d.H₂O (8 mL) and the two solutions combined. The pH was then adjusted to pH 9.1.

*HEPES buffer (10 mM):* HEPES buffer (0.12 g) was dissolved in d.H₂O (50 mL). The pH was then adjusted to pH 7.4 if required.

*Phosphate buffer (10 mM):* PB (86 mg) was dissolved in d.H₂O (50 mL). The pH was then adjusted to pH 7 if required.

*Lateral flow buffer (LF):* sodium dihydrate phosphate monohydrate (34.4 mg) and bovine serum albumin (BSA, 0.1 g) were dissolved in Tween 20 (10 µL of 5.05%) and d.H₂O (20 mL).

### Preparation of Antibody-Nanoparticle Conjugates

SERS active antibody functionalised silica tags: Raman reporters used were PPY and BPE, and these were used to detect *Listeria monocytogenes (L. mono)* and 'all *Listeria* species' respectively.

Note: initially BPE was used to detect *L. mono* but it became apparent when doing the duplex analysis, it was better to change to PPY as it has an isolated peak at 955 cm⁻¹ making it easier to identify the presence of the *L. mono* species.

The antibodies were passively adsorbed onto the silica coated gold tags using the following method: Silica tags (1 mL) and borate buffer (100 µL; 100 mM; pH 9) were mixed together and vortexed. Following this, *Listeria* antibody (10 µL; 1 mg/mL; *L. mono* Ab or polyclonal *Listeria* Ab) was added and the solution shaken for 2 hours. BSA (100 µL; 10%) was then added and the solution shaken for a further 30 mins. Samples were centrifuged (3800g for 20 mins) and then re-suspended in borate buffer (200 µL for lateral flow (LF) analysis; 500 µL for magnetic detection assay or 1 mL for characterisation - extinction spectroscopy, dynamic light scattering (DLS), zeta potential analysis (zeta) and SERS) Note, each time two controls were also prepared, a Si tag only control and a Tag + BSA control.

Testing the SERS active conjugates and controls (Si tag only and Tag+ BSA) across a range of pH's (4-9), the above protocol was followed except instead of using borate buffer at pH 9 the following buffers (same volumes) were used: pH 4.4 - acetate buffer; pH 7 - phosphate buffer or pH 7.4 - HEPES buffer.

Polyclonal *Listeria* antibody functionalised Ag@MNP: These conjugates were also prepared using a passive adsorption approach. Ag@MNP (500 µL) and borate buffer (100 µL; 100 mM; pH 9) were mixed together and vortexed. Following this, polyclonal *Listeria* antibody (20 µL; 1 mg/mL) was added and the solution shaken for 2 hours. BSA (100 µL; 10%) was then added and the solution shaken for a further 30 mins. Samples were centrifuged (3800g for 20 mins) and then re-suspended in borate buffer (200 µL for LF analysis; 500 µL for magnetic assay or 1 mL for characterisation - extinction, DLS, zeta and SERS). Note, each time two controls were also prepared, a Ag@MNP only control and a Ag@MNP + BSA control.

### Bacterial Strains and Growth Conditions

*Bacterial strains used throughout: Listeria monocytogenes (L. mono)* ATCC 15313, *Listeria welshimeri (L. welsh)* ATCC 35897, *Listeria innocua (L. innoc)* ATCC 33090, *Listeria ivanovii (L. ivano)* ATCC 19119, *Listeria seeligeri (L. seelig)* ATCC 35967 and *Escherichia coli (E. coli)* ATCC 25922.

*Listeria* species were grown on BHI agar in an aerobic atmosphere for 24 hours at 37 °C whilst *E. coli was* grown on LB Miller agar under the same conditions. Harvested cells were suspended in either BHI or LB broth to obtain an optical (OD_{600 nm}) of 0.6. Cells were then plated onto BHI or LB agar plates and incubated for 24 hours as described previously. All the strains were grown using the same batch of culturing plates, in order to reduce any potential unwanted phenotypic variation.

### Bacterial Sample Preparation

Bacterial slurries were prepared by harvesting the biomass from the surface of each plate using sterile inoculating loops and re-suspending in physiological saline solution (1 mL; 0.9% NaCl). The prepared bacterial slurries were washed by centrifugation (*E. coli -* 650g for 5 min, *Listeria* species - 1600g for 5 min) and the pellets re-suspended in 1 mL saline. The wash step was repeated a further twice with the final re-suspension being in d.H₂O (1 mL). Note the OD_{600 nm} was recorded for all samples and bacterial concentrations were obtained by serial dilutions based on plate-counting results. All samples were stored at -80 °C until further analysis.

### Lateral Flow Analysis

Lateral flow analysis were used to determine whether the antibodies had successfully attached to the nanoparticle surface. The following protocol was performed for each lateral flow assay: A lateral flow buffer was prepared by adding phosphate buffer (34.4 mg,) BSA (100.0 mg) and Tween 20 (10 µL in 20 mL d.H₂O). The lateral flow buffer (80 µL) and antibody conjugates (20 µL) to be tested were added to a glass vial.

The bacteria (0.5 µL) was then spotted in the middle of a lateral flow strip (nitrocellulose), and anti-mouse IgG (control spot as all antibodies bind if present; 0.5 µL) was spotted near the top of the strip. Once dry, the strip was then placed inside the vial, positioned to be in contact with the liquid but not covering the bacteria spot. The appearance of any spots was then recorded and photographed after 15 minutes once the solution had travelled up the strips fully.

### Detection Assay

The novel method for bacterial detection described previously by Kearns et al.^{5,6} has been modified for use in this study.

Single pathogen detection: For each of the bacterial pathogens the following procedure was carried out - SERS active antibody functionalised Si tags (200 µL; Ab-AgNP) were added together with polyclonal *Listeria* antibody functionalised Ag@MNP (200 µL; poly Ab-Ag@MNP) and a specific bacterial strain (50 µL, various concentrations ranging from 10⁴ to 2 CFU/mL). Note for the control sample, the bacteria was replaced with d.H₂O (50 µL) only or a non-specific species i.e *L. welsh* (50 µL; 10⁴ CFU/mL). The sample was mixed thoroughly via vigorous stirring (1600g) for 5 min before being placed in a magnetic rack for a further 25 min to allow the sample to collect. The clear supernatant was removed and the sample resuspended in d.H₂O (1 mL) ready for analysis. It should be noted that each sample was prepared in triplicate.

Duplex detection: The two sets of SERS active antibody functionalised Si tags (100 µL of each tag) were added together with polyclonal *Listeria* antibody functionalised Ag@MNP (200 µL) plus the two or three *Listeria* strains (50 µL of each pathogen; various concentrations ranging from 10⁴ to 10 CFU/mL). Note for the control sample, the bacteria was replaced with d.H₂O (50 µL) only or a non-specific pathogen i.e. *E. coli* (50 µL; 10⁴ CFU/mL). The same procedure as described above (for the single pathogen detection) was employed.

### SERS and Multivariate Analysis

Raman reporters used were PPY and BPE, and these were used to detect the presence of *L*. *mono* and 'all *Listeria* species'respectively.

The samples were analysed immediately after preparation using a Wasatch Photonics (Morrisville, USA) portable Raman spectrometer with a 633 nm laser excitation. The laser was focussed directly into a 1 mL glass vial containing 1 mL of the antibody-NP conjugate solution. All the measurements had a 0.1 or 1 s acquisition time (stated in figure caption) and a laser power operating at 50 mW. For all Raman spectra, data handling was carried out in Excel software. The peak intensities were obtained by scanning 3 replicate samples 5 times and in all plots the error bars represent one standard deviation.

For the characterisation of the antibody-NP conjugates, the samples were analysed using a SnRI (Snowy Range Instruments, Laramie, USA) portable Raman spectrometer and a laser excitation wavelength of 638 nm. All the measurements had a 1 s acquisition time and a laser power operating at 50 mW. Each sample was prepared in triplicate and 5 scans of each replicate were recorded.

For the analysis of the magnetic plug, the sample was prepared as described in the 'single pathogen detection assay,' but instead of re-suspending the solution and essentially focussing the laser into the 'free solution,' the laser was focussed directly at the magnetic plug. The instrumental settings were the same as those described above. Briefly, 633 nm Wasatch spectrometer with an acquisition time of 1 s.

### Additional materials and methods

The experimental procedures for Fig. 10 - Fig. 23 are the same as above but with additions or modifications as follows.

### Chemicals

Maximum recovery dilutant (MRD) was sourced from Merck. Swabs (namely, NRS II) containing neutralise rinse solution (NRS) were provided by Bradgate Bakery. 10x10 cm swabbing template was sourced from VWR. Tin foil, chicken and bacon sandwiches and cheese and ham sandwiches were sourced from a leading supermarket.

### Preparation of Food Matrix and Listeria-Food Matrix Samples:

The food matrix (FM) was prepared by blending a sandwich (75 g) in MRD (75 mL). Either a chicken and bacon sandwich or cheese and ham sandwich were used as the food matrices (stated in Figure captions).

The Listeria-FM samples were prepared by combining Listeria solution (900 µL; 10⁴ to 5 CFU/mL) with FM (100 µL).

Listeria-FM samples for swabbing were prepared by mixing Listeria solution (1 mL; 10⁵ to 10 CFU/mL) or BHI (1 mL; control) with FM (1 mL) to make 'pre-mixed' samples.

### Swabbing Procedure:

Swabbing was carried out on tinfoil on the bench, within a 10x10 cm area. A cardboard swabbing template was used to map out the required area. L. welsh (Non-pathogenic strain; 1 mL; 10⁵ - 10 CFU/mL) or BHI (1 mL; control) was pipetted into the middle of the template and smeared using a plastic L-spreader. This was left to dry before being swabbed and extracted into NRS solution. The swab was vortexed for 10 secs to release the bacteria into the NRS solution. The NRS-bacteria solution was then analysed by plating (100 µL) on BHI agar and incubating at 37 °C for 24 hr to allow for bacterial counts and also analysed using the magnetic assay (100 µL of NRS-bacteria solution).

Swabbing with FM was carried out in the same manner, except pre-mixed Listeria-FM (1 mL; 10⁵ - 10 CFU/mL) or pre-mixed BHI-FM (1 mL) was smeared onto the tin foil.

*NRS II swabs are used for testing in food environments as they contain blue shafts and can easily be identified if a problem occurs (broken, fall into food etc) whereas, white or clear swabs are not easily identified.

### Detection Assay

**Single pathogen detection:** For each of the bacterial pathogens the following procedure was carried out - SERS active antibody functionalised Si tags (200 µL; Ab-AgNP) were added together with polyclonal Listeria antibody functionalised Ag@MNP (50 µL; poly Ab-Ag@MNP) and a specific bacterial strain (50 µL, various concentrations ranging from 10⁵ to 2 CFU/mL). Note for the control sample, the bacteria was replaced with d.H₂O (50 µL) only or a non-specific species i.e L. welsh (50 µL; 10⁴ CFU/mL). The sample was mixed thoroughly via vigorous stirring (1600g) for 5 min, unless stated otherwise, before being placed in a magnetic rack for a further 25 min (unless stated otherwise) to allow the sample to collect. The clear supernatant was removed and the sample re-suspended in d.H₂O (1 mL) ready for analysis. It should be noted that each sample was prepared in triplicate.

**Duplex detection:** The two sets of SERS active antibody functionalised Si tags (100 µL of each tag) were added together with polyclonal Listeria antibody functionalised Ag@MNP (50 µL) plus the two or three Listeria strains (50 µL of each pathogen; various concentrations ranging from 10⁵ to 10 CFU/mL). Note for the control sample, the bacteria was replaced with d.H₂O (50 µL) only or a non-specific pathogen i.e. E. coli (50 µL; 10⁴ CFU/mL). The same procedure as described above (for the single pathogen detection) was employed.

**FM samples:** The same parameters above were used in the spiked food experiments except the Listeria-FM and control-FM volumes were increased to 500 µL to see the impact the food matrix had on the assay.

**Swabbing:** The same parameters above were used in the swabbing experiments except the bacteria and control volumes were increased to 100 µL to compliment the volumes used in the plating/ bacterial counts. It should be noted that the Listeria concentrations shown in the figures are those of the complementary plating experiment (i.e. extracted from swabs) and not the concentrations of Listeria solutions pipetted directly onto the surface.

### SERS Analysis

Instrumental procedures used herein were the same as those previously provided, with the portable Wasatch Raman spectrometer and acquisition times of 0.1 or 1 s (as stated in figure caption) being used throughout. Except in the experiments where the handheld CBEx Raman spectrometer was used to demonstrate the adaptability of the assay to longer excitation wavelengths; details of which are provided below.

Raman reporters used were PPY and BPE, and these were used to detect the presence of L. mono and 'all Listeria species' respectively.

The samples were analysed immediately after preparation using a CBEx (Snowy Range Instruments, Laramie, USA) handheld Raman spectrometer with a 785 nm laser excitation. All the measurements had a 1 s acquisition time and a laser power operating at 100 mW. For all Raman spectra, data handling was carried out in Excel software. The peak intensities were obtained by scanning 3 replicate samples 5 times and in all plots the error bars represent one standard deviation.

### Results and discussion

The developed bionanosensor is based on a sandwich-type SERS assay with the detection strategy provided in Figure 1. Our approach involves combining magnetic nanoparticles functionalised with a polyclonal *Listeria* antibody that enables the capture and isolation of the *Listeria* species, plus SERS active nanoparticles that are *Listeria* strain specific. These optically bright nanoparticles are also responsible for providing the unique Raman spectrum observed when interrogated with a portable Raman spectrometer.

Test 1 - assay format for *L. mono* detection: i) Polyclonal *Listeria* antibody functionalised silver coated magnetic nanoparticles (Ag@MNPs) will bind to *L. mono* bacteria, and this will allow magnetic separation of the bacteria from the sample matrix ii) SERS active silica capped gold nanoparticles (Si tags) functionalised with a biorecognition molecule *(L. mono* specific antibody (Ab)) and a unique SERS reporter are added. The mixture is shaken vigorously for 5 min before application of a magnet for a further 25 min and collection of the sample. Any unbound matrix is gently removed and the sample subsequently re-suspended in d.H₂O (deionised water). iii) The sample is then interrogated with a 633 nm laser beam and SERS signal obtained. When no target is present the SERS active Si tags will be washed away, thus they won't bind to bacteria so a minimum SERS signal is obtained (black spectrum).

Test 2 *- Listeria* multiplexing: 2× SERS active Si tags each functionalised with a different Raman reporter and an antibody (the first Ab-SERS tag is specific to *L. mono* only and the second is functionalised with a polyclonal *Listeria* Ab) are added together with multiple *Listeria* species and polyclonal *Listeria* functionalised Ag@MNPs (these conjugates bind to all *Listeria* species). In the same way as the single-plex systems, magnetic separation allows for the samples to be concentrated and analysed via a 633 nm laser. A SERS spectrum is obtained which contains characteristic peaks (highlighted with an asterix) from the two Raman reporters and thus can be used to confirm when the targets are present. If the 955 cm⁻¹ peak from PPY is observed, then *L. mono* is present. If not, but there is a peak at 1643 cm⁻¹ for BPE then one of the other *Listeria* species is present and further investigations can be conducted to determine which strain. If no peaks are observed, then no *Listeria* species are present and the signal should be similar to that of the control.

Both sets of nanoparticle conjugates (SERS active and Ag@MNP capture conjugates) were optimised and characterised using extinction spectroscopy, dynamic light scattering (DLS), zeta potential analysis and SERS (SERS active conjugates only). The data obtained at each stage of the conjugation process can be seen in Figure 2. In summary, a small shift in the localised surface plasmon resonance (LSPR) plus broadening of the peak was observed after the addition of the antibodies (Figure 2a and b). Furthermore, an increase in size and decrease in zeta potential (Figure 2c and d, respectively) were observed at each stage of the conjugation indicating that there was a change to the surface environment of the nanoparticle and thus confirming the successful attachment of the antibodies to the nanoparticle's surface. Moreover, the SERS results showed that with a 1 s accumulation time and 633 nm laser an excellent signal could be obtained from the SERS active conjugates (Figure 2e).

***L. mono* Detection Assay.** As explained above, the detection assay involves the use of polyclonal *Listeria* functionalised magnetic nanoparticles and SERS active tags functionalised with antibodies that are bacterial strain specific. It is important to note that the Raman reporter is only present on the biorecognition nanoparticles and not on the magnetic nanoparticles. Hence, a SERS signal is only observed when the SERS active tag binds specifically to its bacterial target. As shown in Figure 3, a SERS signal is only obtained when both the biorecognition nanoparticles and the magnetic nanoparticles bind to *L. mono.* A magnet is then applied, and magnetic separation is used to remove the SERS active complex from the matrix volume. In the case where there was no bacterial target present or a non-specific species was added, the SERS active conjugates were unable to bind and are essentially washed away during the magnetic separation step, leaving only the magnetic nanoparticles to be analysed, thus resulting in a very weak Raman signal being obtained (black spectra).

In terms of the assay's sensitivity, it is obvious from Figure 3 that detection can confidently be made with cell concentrations between 10⁴ and 5 CFU/mL for *L. mono.* The signals for the samples at these concentrations are far superior to the controls. The lowest discrimination between the sample and control, was found to be with a *L. mono* concentration of 5 CFU/mL, with a signal enhancement approximately twice that of the controls.

Moreover, it can be seen that there is a concentration to signal enhancement effect, such that greater intensities of signal are observed with increasing bacterial concentrations. This allows quantification of a bacterial concentration to be determined, particularly when control samples (with known concentrations of bacteria are used as comparators) or calibration testing has been carried out, so as to permit subsequent tests to be compared against calibration values.

***Listeria* Multiplex Detection.** For the multiplex detection assay as shown in Figure 1-test 2, the same procedure described previously was followed except two separate sets of SERS active tags were prepared, each with a different Raman reporter. The first incorporates the Raman reporter, PPY and contains antibodies which are specific to *L. mono* only and the second is capped with BPE and is functionalised with polyclonal *Listeria* antibodies which will bind to all *Listeria* species. In the same way as the single-plex system, the SERS active conjugates are added together along with the *Listeria* species and magnetic nanoparticles, and the 'bacterial complex' is formed. Magnetic separation is performed and the samples are analysed using a portable Wasatch 633 nm Raman spectrometer. If *Listeria* is present, then an intense SERS spectrum will be obtained which contains characteristic peaks (highlighted with an asterix in Figure 1 -test 2) from the Raman reporters and thus can be used to confirm which *Listeria* species are present. If the 955 cm⁻¹ peak from PPY is observed, then *L. mono* is present. If not, but there is a peak at 1643 cm⁻¹ for BPE then one of the other *Listeria* species is present and further investigations can be conducted to determine which strain. If no peaks are observed, then no *Listeria* species are present and the signal should be weak and similar to that of the control.

Figure 4a shows the SERS spectrum obtained when multiple *Listeria* species (*L. mono, L. welsh* and *L. innoc*) were present in a sample and also when *L. mono* was absent but other *Listeria* species were present. The multiplex spectra with *L. mono* present was compared to the multiplex spectrum with *L. mono* absent but other *Listeria* species present (*L. welsh* and *L. innoc*); to the SERS spectra obtained when *L. mono* (reference spectrum for PPY) and *L*. *welsh* (reference spectrum for BPE) were detected individually; to control samples for when no bacteria was present and also when non-specific bacteria (*E. coli*) was added instead.

It can be seen in Figure 4a that the black dotted line highlights two peaks at 955, and 1643 cm⁻¹ which are unique to each of the Raman reporters (PPY and BPE) and hence used to identify the presence of *L. mono* and 'all *Listeria* species' respectively. In the top two spectra, the 1643 cm⁻¹ peak of BPE is observed confirming that *Listeria* is present. The 955 cm⁻¹ peak of PPY is also visible in these two spectra, hence it can be confirmed that *L. mono* is present and is one of the *Listeria* species being identified. Again, in the third spectrum (down from the top) 1643 cm⁻¹ peak of BPE is observed confirming that *Listeria* is present, however, the 955 cm⁻¹ peak of PPY is not visible and hence it can be confirmed that *L. mono* is not present in this sample. The fourth spectrum is a reference for the presence of *L. mono* and the spectrum of PPY is solely observed. The fifth spectrum is a reference for the presence of *'Listeria* species' and peaks solely relating to BPE are observed. The spectra for the control samples are very weak and hence confirms that when no *Listeria* species are present or non- specific bacteria are added instead, then the SERS active conjugates do not bind and are washed away during the magnetic separation, resulting in weak spectra being obtained. This is also confirmed in Figures 4b and c) where a bacterial concentration study for the *Listeria* multiplex and the associated signal enhancements are shown.

It can be observed in Figures 4b and c) that the multiplex assay gives clear discrimination between samples and controls at all bacterial concentrations. Even at the lowest bacterial concentration of 10 CFU/mL, the signal enhancement for the sample is ~4 times greater than that of the control. Furthermore, both the 955 cm⁻¹ peak from PPY and 1643 cm⁻¹ peak from BPE, are clearly visible at all concentrations, hence confirming that the assay is able to detect the presence of *L. mono* in a complex matrix down to the sensitive detection of 10 CFU/mL. This bionanosensor provides a "one pot" detection system for *Listeria,* but more importantly it is capable of isolating and discriminating between when *L. mono* is present or absent.

The above teaching provides an overall assay time of 30 minutes and allows sensitive detection of *L. mono* at concentrations in a single-plex assay at 5 CFU/mL and 10 CFU/mL for *Listeria* species in a complex matrix. However, it is believed that this can be significantly reduced, such as by reducing mixing time and time for magnetic separation. It is envisaged that, with optimisation, it is possible to have an overall time of 15 mins from taking a sample to producing a result.

**Magnetic Plug vs Free in Solution.** Magnetic nanoparticles (MNPs) have shown promise in a number of biomedical applications.⁷ To date, most assays that incorporate magnetic separation steps, use magnetic nanoparticles to capture and isolate biological targets. Magnetic separation steps are very useful as they enable unbound materials to be removed/washed away and also for samples to be concentrated before detection occurs. We also considered relaxing of the magnetic force following a washing step and re-suspending the sample back into solution so the bacterial-complex is essentially 'free in solution' leading to an increased signal with reproducible results. Figure 5, compares the results for the single-plex detection of *L. mono* when the laser is directed straight at the magnetic plug versus when the sample is free in solution.

It is clear from Figure 5a that when the sample is free in solution a greater signal is obtained than when the magnetic plug is interrogated, it's ~10 times greater. Also, we can see from Figure 5b, that the signal obtained from the magnetic plug is not as reproducible and a large interference background is observed. Hence interrogating the sample when it's 'free in solution," gives much more intense results with better reproducibility.

***L. mono* Detection Assay across pH range 4-9.** Usually acidic conditions are used to passively adsorb antibodies onto NP surfaces due to electrostatic forces. However, for our technology these conditions do not work and hence in order to achieve the best SERS results and successfully attach the antibody to the Si surface we have to pH correct to 9.1 (basic conditions).

Before the *L. mono* assay was conducted at different pH's, it was important to determine the stability of the Si tags at each step of the conjugation process. Hence, for each of the different buffers 3 samples were prepared; Si tag only, Tag + BSA, Tag + BSA + *L. mono* Ab*.* The buffers with associated pH values were as follows: acetate buffer - pH 4.4, PB buffer - pH 7, HEPES buffer - pH 7.4 and borate buffer - pH 9.1. For characterisation purposes, extinction spectroscopy, DLS, zeta potential and SERS analysis was conducted on each of the samples and the results for the controls i.e. Si tag only and Tag + BSA can be seen in figures 6 and 7 respectively.

It can be observed across all the characterisation tests in Figure 6, that the Si tags in acetate buffer (pH 4) are very unstable. Firstly, in Figure 6e, it can be seen that the Si tags have precipitated out of solution, indicating that the nanoparticles have significantly aggregated and that they are unstable in this acidic environment. This sample was vigorously shaken back into solution in order for spectroscopic analysis to be conducted. Significant dampening and broadening is observed in the extinction spectrum indicating that the tags are unstable and likely aggregating, this is further confirmed with a large increase in size and decrease in the zeta potential. The SERS signal (6d) is intense but this is common for severely aggregated nanoparticles. Hence it can be concluded that these Si tags are unstable in this acidic buffer. For the Si tags in the neutral and basic buffers (pH 7 to 9 respectively), the nanoparticles remain stable with minimum or no aggregation being observed. It can be seen in Figure 6e, that the samples were easily re-suspended back into solution, there was little difference in their extinction profiles, size or zeta potential values and all gave intense SERS spectra, indicating their stability in these buffer environments and at these pH's.

Similar results were observed for the Tag + BSA samples (Figure 7a-d), with the pH 4 sample in acetate buffer precipitating out of solution again and the characterisation tests showing that the sample had aggregated and become unstable (decrease and broadening in extinction profile, increase in size, decrease in zeta, intense SERS). Again the Tag + BSA samples in the neutral and basic buffers indicated the samples were stable with only slight changes being observed in their extinction profiles, size and zeta measurements. However, there was a noticeable difference in the SERS spectrum for the Tag + BSA in PB buffer (pH 7) with a large background at ~ 1200 cm⁻¹ appearing. It is believed that this is a result of an interaction occurring between the BSA and PB buffer and could cause a problem if these conditions were to be used in the *L. mono* detection assay.

Before conducting the *L. mono* assay at the different pH's, it was important to further confirm that the antibodies had successfully attached to the Si tags at the different pH's and so lateral flow (LF) analysis was conducted (see Figure 8). A LF assay is a nitrocellulose paper-based platform for the detection and quantification of analytes in mixtures. Liquid samples containing the analyte of interest (in this case, the antibody-nanoparticle conjugates) move through various zones of the polymeric strips via capillary flow, on which molecules that can interact with the analyte are attached.⁸ The bacteria of interest and anti-mouse IgG (control) were attached to the strips and the capture of the bacteria by the nanoparticle conjugates was confirmed by the appearance of spots.

Following on from the characterisation experiments of the controls, it was no surprise to see that for the sample in acetate buffer (at pH 4) there were no spots observed for either the IgG control or any bacterial strain. This confirms that the *L. mono* antibody does not attach to the Si tag surface at this pH and further indicates that these nanoparticles are not stable in these acidic conditions and hence should not be used in the *L. mono* detection assay.

Although the control samples in HEPES buffer (pH 7) looked stable and promising, it appears from the LF analysis that the antibody is weakly attached to the surface of the Si tag, if at all. Very weak spots were observed with the IgG control and possibly a very weak spot can be seen with *L. mono* at 10⁴ CFU/mL. However, a spot was not observed at the higher *L. mono* concentration (10⁸ CFU/mL). It appears that only a small amount of antibody has bound to the nanoparticle surface, hence it is unlikely that there will be enough antibody present for these conjugates to bind and capture *L. mono* in the detection assay.

For the Tag + *L. mono* Ab in PB buffer (pH 7), two spots are clearly observed, one for the IgG control and the second for the *L. mono* (bacteria) spot. This can be seen at both the *L. mono* bacterial concentrations (10⁸ and 10⁴ CFU/mL), confirming that the antibody is present on the silica surface and that these conjugates are specific and will bind to *L. mono.* Furthermore, a spot is observed for the IgG control on both the *L. welsh* and *E. coli* strips but no spots are observed for where the non-specific bacteria was spotted, confirming that the antibody is present but it does not bind to these strains., thus demonstrating the specificity of the antibody towards *L. mono.* Similar results were observed at pH 9, for the Tag + *L. mono* Ab in borate buffer. The spots observed at pH 9 are slightly brighter than at pH 7 in PB, suggesting that the binding could be stronger but both sets of conjugates show promising results for use in the *L*. *mono* detection assay.

The detection of *L. mono* across the pH range 4-9, with the associated signal enhancement values calculated are shown in Figure 9.

At pH 4, all the data collected supports the theory that the Si tags are not stable and hence the assay cannot be performed using these acidic conditions. Not only are the tags unstable but the antibody does not bind when using this buffer and this is further confirmed in Figure 9a, where the Tag + *L. mono* Ab sample gives a similar intensity to the control. Therefore, confirming that the conjugates do not bind to the *L. mono* bacteria and are simply washed away during the magnetic separation step. Thus, this assay cannot be performed in this acidic environment. It should be noted that the slight increase in signal intensity for the sample and control at this pH when compared to the other conditions, is likely due to the particles being unstable and aggregating. An increase in signal due nanoparticles aggregating is commonly observed in SERS.

At pH 7 in HEPES (figure 9c), the *L. mono* assay is poor with only a slight increase in signal from the sample over the control, thus confirming that the antibody is only weakly bound to the nanoparticle surface. It is unlikely that enough antibody is present to capture the *L. mono* bacteria and form a bacteria-complex, thus most of the SERS active conjugates are being washed away during the magnetic separation step, resulting in a poor signal being obtained. Hence these conditions and this buffer cannot be used for *L. mono* detection.

Good discrimination between sample and control is observed at pH 7 in PB buffer and at pH 9 in borate buffer, Figures 9b and 9d, respectively. Hence for these samples, the bacteria-complex has successfully formed, with unbound material being removed during the magnetic separation step, and when interrogated with the 633 nm laser strong SERS results have been obtained. Signal enhancements of ~ 8 times and ~13 times in PB and borate respectively are observed for the samples over the controls. It should be noted however, that the background inference observed when BSA interacts with PB (Figure 7d) is also observed again in Figure 9b, for *L.* mono detection in PB and this could be a problem at lower bacterial concentrations. Furthermore, the signal obtained in PB is lower (by ~5000 au) than that observed with borate buffer at pH 9, hence performing the assay in basic conditions appears to give the best results with minimal background inference and greatest SERS enhancement being obtained. The Si tags and conjugates are stable in these basic conditions and across all the characterisation experiments, the conjugates and controls performed best at this pH with the most reproducible and strongest SERS signals being obtained. Therefore, it can be concluded that using borate buffer and basic conditions for our technology gives the best results.

### L. mono Detection Assay

As explained previously (in Figure 1 and 3), the detection assay involves the use of polyclonal Listeria functionalised magnetic nanoparticles and SERS active tags functionalised with antibodies that are bacterial strain specific. It is important to note that the Raman reporter is only present on the biorecognition nanoparticles and not on the magnetic nanoparticles.

Hence, a SERS signal is only observed when the SERS active tag binds specifically to its bacterial target. As shown in Figure 10, a SERS signal is only obtained when both the biorecognition nanoparticles and the magnetic nanoparticles bind to *L. mono.* A magnet is then applied, and magnetic separation is used to remove the SERS active complex from the matrix volume. In the case where there was no bacterial target present or a non-specific species was added the SERS active conjugates were unable to bind and essentially washed away during the magnetic separation step, leaving only the magnetic nanoparticles to be analysed, thus resulting in a very weak Raman signal being obtained.

Following on from previous studies, it was important to do a *L. mono* concentration study that covered an extensive range thus allowing a limit of observation (LOD) to be accurately determined.

Previous studies, allowed for 5 CFU/mL to be detected in the single pathogen tests. However, it is obvious from Figure 10, that detection can confidently be made with cell concentrations over a greater range, from 10⁵ to 4 CFU/mL for *L. mono.* The signals for the samples at these concentrations are far superior to the controls. The lowest discrimination between the sample and control, was found to be with a *L. mono* concentration of 4 CFU/mL, with a signal enhancement approximately 1.7x that of the controls.

Moreover, it can be seen that there is a concentration to signal enhancement effect, such that greater intensities of signal are observed with increasing bacterial concentrations. This allows quantification of a bacterial concentration to be determined, particularly when control samples (with known concentrations of bacteria are used as comparators) or calibration testing has been carried out, so as to permit subsequent tests to be compared against calibration values. Figure 10c, further confirms this concentration to signal effect with a linear relationship being observed over the bacterial concentration range 10⁵ to 10 CFU/mL, which is equivalent to 5 to 1 respectively on the log scale. The equation of the line allows for LODs to be calculated and unknown samples to be quantified. [9]

### L. mono Detection in a Food Matrix

It was important to investigate if the assay could be used for testing in complex systems. One of the areas of interest is testing for Listeria in the chilled food environment, hence a food matrix such as a blended sandwich was used to mimic a complex system. In our studies, we used a blended chicken mayonnaise sandwich as we were advised by industry leaders that this was the most popular sandwich choice in the UK at the time of testing. *L. mono* with bacterial concentrations from 10⁴ to 5 CFU/mL were spiked into our complex food matrix and analysed. It can be seen from Figure 11, that detection can confidently be made with cell concentrations between 10⁴ and 10 CFU/mL for *L. mono.* The signals for the samples at these concentrations are far superior to the controls, even though a larger background was observed. This larger background is attributed to the complex food matrix. Even at 5 CFU/mL, discrimination between the sample and the controls can be observed with a signal enhancement of approximately 1.4x. It should be noted that the magnetic separation step is a critical component in our assay and it is due to this separation and the ability to extract the 'bacterial complex' whilst washing away unbound materials, even components of food, that the large discriminations between samples and controls are permitted.

### Swab Extraction and Detection of Listeria

Many testing environments involve using swabs to extract bacteria from surfaces, hence it is essential that we can incorporate this procedure into our assay whilst still being able to detect *L. mono* at a range of concentrations and without compromising our assay's performance or sensitivity. Figure 12, demonstrates the ability to swab Listeria (*L. welsh* was used in these studies as it a non-pathogenic strain) from a surface and detect it in our SERS assay, while Figure 13, demonstrates the ability to detect Listeria from a surface that has an interferent present such as food components. In this case, the surface had both Listeria and components from a blended chicken mayonnaise sandwich present. It should be noted that the Listeria concentrations seen in the figures are those of the complementary plating experiments (i.e. extracted from swabs) and not the concentrations of Listeria solutions or pre-mixed Listeria-FM solutions, pipetted directly onto the surface.

It can be seen in Figure 12, that the extracted Listeria concentrations from the swabs, ranged from 10⁵ to 60 CFU/mL and subsequently these were the concentrations which were analysed in the SERS assay. It is obvious from Figure 12, that detection can confidently be made across all the Listeria concentrations tested, with the lowest detection at 60 CFU/mL demonstrating a signal enhancement of 4.4x for the sample over the control. It should be noted that the signals obtained in Figure 12 were comparable (in similar range) to those seen previously in Figure 10, where Listeria was added directly to the sample and analysed, hence it allowed us to predict the concentrations of those >10³ CFU/mL where the bacterial counts where too high to count on the complementary plating experiments.

In Figure 13, food components were added along with Listeria to the surface, swabbed, extracted and then analysed using the SERS assay over the bacterial concentration range 10⁴ to 1 CFU/mL. Successful detection of the Listeria was achieved for the concentration ranges 10⁴ to 38 CFU/mL, with signals for the samples being far superior to the control. 1 and 0 CFU/mL samples gave signals similar to that of the control sample, but this was to be expected based on previous studies (Figure 10) where the LOD for this assay is 4 CFU/mL, hence these samples are below this threshold.

The ability to incorporate swab extraction into our SERS assay, with and without an interferent being present, is critical for our technology and we have successfully demonstrated this without compromising our assay's performance.

### Improving Assay Time

The overall time of the assay is currently 30 minutes from taking a sample to producing a result, however several optimisation steps have been taken to improve this and make the assay significantly faster. The first optimisation step as demonstrated in Figures 14 and 15, involved reducing the mixing time; this is the length of time required for the biological interaction to occur between the bacteria and the antibody-nanoparticle conjugates (Ab conjugates). The current mixing time is 5 mins and involves vigorous shaking (at 3000 rpm), therefore, a time study was performed to determine if this mixing time could be reduced. As shown in Figure 14, the SERS assay was performed with mixing times from 5 mins to 5 secs using a vortex. For mixing times below 1 min the vortex was set at 3000 rpm and the eppendorf (containing the samples/controls) held in place, but for samples with mixing times of 1 and 5 mins these eppendorfs were placed on a shaker with a speed of 3000 rpm.

It can be seen from Figure 14, that successful detection was achieved for all the times trialled. The results also indicate that the current mixing conditions at 5 mins is in fact too long, with the SERS signal being weakest when compared to all the other times. This increased time is likely allowing for more non-specific interactions to occur, however the effect is minimised by reducing the mixing time. The optimum mixing time is actually 30 secs but excellent discrimination between sample and control can be achieved in only 5 secs, with a signal enhancement of 20.1x being obtained.

Another important factor to consider when optimising the SERS assay, was to investigate whether vigorous shaking (using a vortex) had to be implemented into our system or whether hand-mixing of the samples would be sufficient to allow the biological interaction to occur. Figure 15, shows a second time study, with samples being mixed by hand, for 30 secs to 5 secs. All mixing times showed greater signal enhancements than previously observed with 5 mins of mixing and again the optimum time for mixing the bacteria with the NPs was 30 secs. The signal enhancement with hand mixing for 30 secs was actually greater than that observed with the vortex. This effect was also seen with the 15 secs and 10 secs samples, however with only 5 secs of hand-mixing the vortex sample gave a greater signal enhancement. The signal enhancement for the 5 secs of hand-mixing was still high however, and significantly greater than the current 5 min mixing conditions. With a 17.5x discrimination between sample and control we can confidently detect *L. mono* with this 5 secs sampling methodology. Furthermore, this improvement could not only make the assay faster but if mixing can be performed by hand it makes it more user-friendly and cost-effective as a mixing device is not required to be implemented into the technology.

In a complementary study, the magnetic separation step was investigated as it is the other time critical component in the SERS assay. The magnetic separation step is currently 25 mins, and this is the time it takes to capture the 'bacterial complex' from a sample matrix. As mentioned previously, the magnetic separation is critical in that it allows the bacterial complex to be collected, whilst washing away unbound materials. Figure 16, shows a time study that investigates if it's possible to reduce the magnetic separation time, without compromising the SERS signals. A time study was performed with the magnetic separation time being reduced from 25 mins to 5 mins, however the mixing time remained at 5 mins.

As shown in Figure 16, 25 mins gave the strongest SERS signal and the biggest signal enhancement (14.9x) with 5 mins giving the weakest SERS signal and lowest signal enhancement (9.1x). Hence, it can be deduced that the longer the samples are left to collect on the magnet, the greater the discrimination between samples and control. However, even at 5 mins there is still good discrimination and confidence in the result, that *L. mono* can be detected using this reduced separation time, thus significantly improving the speed of the assay.

The most important optimisation test was to confirm if the assay could be performed with both a reduced mixing time and reduced magnetic separation time (Figure 17). In addition, it was also essential to determine the importance of mixing and whether it was required for the biological interaction (between Ab-conjugates and bacteria) to occur, hence a 5 sec hand mixing time point was investigated along with a 0 sec time point, which entailed no mixing. A magnetic separation of 5 mins was implemented for both samples.

In Figure 17, detection can confidently be made for *L. mono* with 5 secs of hand mixing. The signal for the sample is far superior to the control. At 0 secs, the signal is significantly weaker than 5 secs, thus confirming that the mixing step is essential and required to be implemented into the assay. These optimisation steps have been critical for improving the assay time; the technology now has an overall assay time of 6 mins (including handling steps) which is a huge advancement from 30 mins and a significant improvement for Listeria testing.

It was important however to test these optimised conditions, with lower Listeria concentrations and with swabbing incorporated into the system, in the presence and absence of a food matrix to ensure the assay's performance was not comprised with the reduced magnetic separation and mixing times. Figures 18-20 show the results from these studies. Figure 18, demonstrates a *L. mono* concentration study over the range 10⁴ to 5 CFU/mL, and it can be seen from the graphs that detection can confidently be made down to 10 CFU/mL, with a signal enhancement of 3.1x for the sample over the control. Even at 5 CFU/mL, the SERS signal for the sample is slightly greater than the control (1.3x) but we are confident that by increasing the mixing time to 10 secs (based on results from Figure 16), will significantly improve the SERS signal and thus, the discrimination between the sample and control. Therefore, allowing for comparable sensitivity (4 CFU/mL) to that achieved previously to be obtained (Figure 10), but with a significantly reduced assay time of just 6 mins.

Figure 19, is a replicate experiment to that shown in Figure 12, except the reduced assay conditions were utilised i.e. 6 mins assay time, involving 5 sec mixing plus 5 mins magnetic separation. It can be seen in Figure 19, that the extracted Listeria concentrations from the swabs, ranged from 10³ to 7 CFU/mL and subsequently these were the concentrations which were analysed in the SERS assay. Successful detection was achieved for all the Listeria concentrations tested, with the lowest detection at 7 CFU/mL demonstrating a signal enhancement of 1.5x for the sample over the control. The control signal has a larger background than previously observed; this is likely due to autofluorescence from biological components in the sample or self-absorption of magnetic NPs that are not removed as effectively with the reduced magnetic separation time. However, the peak at 955 cm⁻¹ which is used to compare the samples to the control and used to calculate the peak heights, is significantly lower for the control than for the samples, regardless of the background, hence the comparison in SERS signal (and signal enhancements) can still be made in the same manner.

It should be noted that the signal enhancements reported in this study, are slightly lower than previously seen with the 30 mins assay (Figure 12), suggesting that some of the 'bacterial complexes' formed are not captured with the reduced time, however, clear discrimination can still be made between samples and controls, therefore, the loss in signal is minimum when compared to the time saved. Moreover, when being compared to the previous study, a lower concentration was detected herein, with just 7 CFU/mL being detected in comparison to 60 CFU/mL. We are confident that using both methodologies (30 and 6 mins assays) however, would enable detection similar to the LOD for the assay (4 CFU/mL - Figure 10) but as previously mentioned, the faster the assay can be conducted, the better the technology becomes and more cost-effective for the end user.

Figure 20, is a replicate experiment to that shown in Figure 13, except the 6 mins assay conditions were employed. The assay was performed over the extracted bacterial concentration range 10³ to 2 CFU/mL, with successful detection being achieved for the concentration ranges 10³ to 54 CFU/mL, with signals for the samples being far superior to the control. The 2 CFU/mL sample gave a SERS signal similar to that of the control, but this was to be expected as it is below the LOD threshold for this assay. The signal enhancements are comparable (just slightly lower) to those reported previously for the 30 mins assay (Figure 13), hence we are confident that we could detect Listeria concentrations <50 CFU/mL, using our 6 mins set-up in the presence of an interferent.

In summary, the present disclosure provides a rapid 'on-site' detection platform for bacterial pathogens, which can give an outcome of the presence/absence of bacteria in just 6 minutes (this includes the manual handling steps - extracting bacteria from swabs, pipetting, mixing, removal of unbound materials etc).

The next Figures presented, all use the 30 mins assay sampling conditions as the work was carried out prior to optimising the magnetic separation step and mixing conditions. Figure 21, is the Listeria multiplex over a greater concentration range than in Figure 4. The multiplex allows for Listeria species to be detected but more importantly it can discriminate when *L*. *mono* is present or absent in a sample.

### Listeria Multiplex Detection.

The same experiment as described previously in Figure 4 was repeated except it was important to do a Listeria concentration study that covered an extensive range thus allowing a LOD to be accurately determined. Previous studies, allowed for 10 CFU/mL to be detected in the duplex test, however it can be seen from Figure 21, that detection can confidently be made with cell concentrations over a greater range, from 105 to 6 CFU/mL, for Listeria species (1643 cm⁻¹ peak present) and L. mono (955 cm⁻¹ peak present). It can be observed in Figures 21 a and b) that the multiplex assay gives clear discrimination between samples and controls down to the sensitive detection of 6 CFU/mL. Furthermore, both the 955 cm⁻¹ peak from PPY and 1643 cm⁻¹ peak from BPE, are clearly visible at all concentrations, hence confirming that the assay is able to detect the presence of *L. mono* in a complex matrix. As explained previously, there is a linear relationship between signal and bacterial concentration and this is illustrated again in Figure 21c, with a linear graph being obtained over the concentration range 10⁵ to 10 CFU/mL which is equivalent to 5 to 1 respectively on log the scale. The equation of the line allows for LODs to be calculated and unknown samples to be quantified. This bionanosensor provides a "one pot" detection system for Listeria, but more importantly it is capable of isolating and discriminating between when *L. mono* is present or absent.

### Portability and Adaptability to Longer Excitation Wavelengths.

To date, the Listeria detection assays (single pathogen and multiple Listeria species) have been performed using a portable Raman spectrometer with a 633 nm laser excitation. Although it is regarded as a portable system, it is quite bulky and requires a connection to a power supply and computer, and therefore cannot easily be employed in a 'realistic field' setting such as at a patient's bedside or food manufacturing site. However, recent advancements in instrumentation has led to the development of low-cost, high-quality, handheld Raman spectrometers that are portable and still able to detect trace amounts of materials. Moreover, they are unique in that they are small, light, battery operated (if required), cost effective and can easily be used by a single operator in diverse and challenging environments.[10] It was important to test our technology using a handheld Raman spectrometer and determine if the sensitivity and selectivity of the system is maintained, whilst using an instrument that could be used for 'on-site' testing (Figures 22 and 23). The handheld instrument chosen to conduct this testing was a CBEx Raman spectrometer with a 785 nm laser excitation. There are additional benefits of using longer excitation wavelengths than those in the visible region, such as reduced background fluorescence/ autofluorescence, limited photobleaching, and the infrared region (>750 to 2500 nm) provides an uncongested spectral window for optical analysis due to the absorption and scattering backgrounds of many molecules (in particular biomolecules) being at a minimum. [9, 11] Figures 22 and 23 replicate the experiments shown in Figure 3 and Figure 4 respectively and are similar to those in Figures 10 and 21 herein, except that a handheld Raman spectrometer with a 785 nm laser excitation was employed instead.

For the single pathogen test, Figure 22, the *L. mono* concentration range tested was 10⁴ to 5 CFU/mL, with successful detection being achieved for all concentrations. The signals for the samples at these concentrations are far superior to the controls. The lowest discrimination between the sample and control, was found to be with a *L. mono* concentration of 5 CFU/mL, with a signal enhancement approximately 2.4x that of the control. Thus, the sensitivity and selectivity of the assay is comparable to that demonstrated with the 633 nm laser excitation (Figure 10), with the added benefit that the background SERS signal commonly observed from the bacteria, is significantly reduced using this longer excitation wavelength.

For the multiplex detection assay, Figure 23, the Listeria concentration range tested was 10⁴ to 5 CFU/mL. It is obvious from Figure 23, that detection can confidently be made across the bacterial range tested with the signals for the samples at these concentrations being far superior to the control. The lowest discrimination between the sample and control, was found to be with a Listeria concentration of just 5 CFU/mL, with a signal enhancement approximately 2.3x that of the control. Again, the performance of the assay is comparable if not slightly better with detection confidently being made at 5 CFU/mL whereas with the 633 nm excitation it was 6 CFU/mL (Figure 21). Furthermore, both the 955 cm⁻¹ peak from PPY and 1643 cm⁻¹ peak from BPE, are clearly visible at all concentrations, hence confirming that the assay is still able to detect the presence or absence of *L. mono* in a complex matrix. Figures 22 and 23, demonstrate the adaptability of the assays for use with longer excitation wavelengths and also the portability of the technology for use in a 'realistic field' setting through the use of a handheld Raman spectrometer. The overall performance of the assay has been maintained, with sensitive and selective detection of *L. mono* and Listeria species being made possible in a single test.

### References

1. J. Turkevich, P. C. Stevenson, J. Hillier, Discuss. Faraday Soc., 1951, 11, 55-75.
2. J. F. Li, X. D. Tian, S. B. Li, J. R. Anema, Z. L. Yang, Y. Ding, Y. F. Wu, Y. M. Zeng, Q. Z. Chen, B. Ren, Z. L. Wang and Z. Q. Tian, Nat. Protoc., 2013, 8, 52-65.
3. G. P. Kumar, N. Rangarajan, B. Sonia, P. Deepika, N. Rohman, C. Narayana, Bull. Mater. Sci., 2011, 34, 207-216.
4. T. Donnelly, W. E. Smith, K. Faulds, D. Graham, Chem. Commun., 2014, 50, 12907-1291.
5. H. Kearns, R. Goodacre, L. E. Jamieson, D. Graham, K. Faulds, Anal. Chem., 2017, 89 (23), 12666-73.
6. C. Rathmell, H. Kearns, D. Graham, L. J. Jamieson, K. Faulds, Spectroscopy (online)., 2019, 34 (6), 24-31
7. E. M. Materon, C. M. Miyazaki, O. Carr, N. Joshi, P. H. S. Picciani, C. J. Dalmaschio, F. Davis, F. M. Shimizu, Appl. Surf. Sci., 2021, 6, 100163.
8. S. S.Agasti, S. Rana, M-Hwan, P. Chae, K. Kim, C-C You, V. M. Rotello,, Adv. Drug Deli. Rev., 2010, 62, 316-328
9. Bedics, M., Kearns, H., Cox, J., Mabbott, S., Ali, F., Shand, N., Faulds, K., Benedict, J., Graham, D., and Detty, M. (2015). Extreme Red Shifted SERS Nanotags, Chem. Sci., 6, pp. 2302 - 2306.
10. Kearns, H., Ali, F., Bedics, M. A., Shand, N. C., Faulds, K., Detty, M. R., and Graham, D. (2017). Sensitive SERS nanotags for use with a hand-held 1064 nm Raman spectrometer, R. Soc. Open Sci, 4, pp. 170422.
11. Culha, M., Cullum, B., Lavrik, N., and Klutse, C. K. (2012). Surface-enhanced Raman scattering as an emerging characterization and detection technique, J. Nanotechnol., 2012, pp. 971380.

## Claims

1. A sandwich immunoassay kit for use in detecting and optionally quantifying *Listeria* in a sample, the immunoassay kit comprising:
a) a first *Listeria* specific antibody, or antigen binding fragment thereof, immobilised to a
first surface, wherein the first surface comprises the surface of a magnetic particle, paramagnetic particle, or nanoparticle; and
b) a second *Listeria* specific antibody, or antigen binding fragment thereof, immobilised to a surface of a particle or bead, wherein the particle or bead further comprises a SERS or a Raman label or dye,
wherein the first and second *Listeria* specific antibodies, or antigen binding fragments thereof, are capable of binding to distinct antigens on the surface of a *Listeria* bacterium, in order to form a detectable complex comprising a *Listeria* bacterium sandwiched between said first and second *Listeria* specific antibodies, wherein the first *Listeria* specific antibody, or antigen binding fragment thereof, is capable of binding any *Listeria* species and the second *Listeria* specific antibody, or antigen binding fragment thereof, is capable of binding a specific *Listeria* species.

2. The kit for use in detecting *Listeria* according to claim 1, wherein the one or more *Listeria* species to be detected is selected from *L. monocytogenes, L. aquatica, L. booriae, L. cornellensis, L. costaricensis, L. goaensis, L. fleischmannii, L. floridensis, L. grandensis, L. grayi, L. innocua, L. ivanovii, L. marthii, L. newyorkensis, L. riparia, L. rocourtiae, L. seeligeri, L. thailandensis, L. valentina, L. weihenstephanensis,* and *L. welshimeri.*

3. The kit for use in detecting *Listeria* according to any preceding claim, wherein the *Listeria* species comprises *L. monocytogenes* and optionally, discerned from one or more further *Listeria* species.

4. The kit for use in detecting *Listeria* according to any preceding claim, wherein the first surface further comprises a coating matrix of a polymer(s), metal, silica or hydroxyapatite.

5. The kit for use in detecting *Listeria* according to any preceding claim, wherein more than one second *Listeria* specific antibody or antigen binding fragment thereof is provided, wherein each said second *Listeria* specific antibody, or antigen binding fragment thereof, is specific for a particular species.

6. A sandwich immunoassay method for use in detecting and optionally quantifying *Listeria* in a sample, the said immunoassay method comprising:
a) providing
i) a first *Listeria* specific antibody, or antigen binding fragment thereof, immobilised to a first surface, wherein the first surface comprises the surface of a magnetic particle, paramagnetic particle, or nanoparticle;
ii) a second *Listeria* specific antibody, or antigen binding fragment thereof, immobilised to a surface of a particle or bead, wherein the particle or bead further comprises a SERS or a Raman label or dye; and
iii) a liquid comprising a sample to be analysed;
wherein the first and second *Listeria* specific antibodies, or antigen binding fragments thereof, are capable of binding to distinct antigens on the surface of a *Listeria* bacterium,
in order to form a detectable complex comprising a *Listeria* bacterium sandwiched between said first and second *Listeria* specific antibodies, and wherein the first *Listeria* specific antibody, or antigen binding fragment thereof, is capable of binding any *Listeria* species and the second *Listeria* specific antibody, or antigen binding fragment thereof, is capable of binding a specific *Listeria* species;
b) permitting the first and second *Listeria* specific antibodies, or antigen binding fragments thereof and the liquid to come into contact for a period of time, in order that any detectable complexes may form between the first *Listeria* specific antibody, or antigen binding fragment thereof, the second *Listeria* specific antibody, or antigen binding fragment thereof and any *Listeria* present in the liquid; and
c) detecting any complexes which are formed, in order to detect and optionally quantify any *Listeria* in the sample.

7. The method according to claim 6, wherein the first *Listeria* specific antibody, or antigen binding fragments thereof, are immobilised to the surface of magnetic or paramagnetic particles, and any complexes are aggregated using a magnetic force prior to detecting any complexes which are formed.

8. The method according to claims 6 to 7, wherein the *Listeria* species to be detected is selected from *L. monocytogenes, L. aquatica, L. booriae, L. cornellensis, L. costaricensis, L. goaensis, L. fleischmannii, L. floridensis, L. grandensis, L. grayi, L. innocua, L. ivanovii, L. marthii, L. newyorkensis, L. riparia, L. rocourtiae, L. seeligeri, L. thailandensis, L. valentina, L. weihenstephanensis,* and *L. welshimeri.*

9. The method according to claims 6 to 8, wherein the *Listeria* species comprises *L*. *monocytogenes* and optionally discerned from one or more further *Listeria* species.

10. The method according to claims 6 to 9, wherein the first surface further comprises a coating matrix of a polymer(s), metal, silica or hydroxyapatite.

11. The kit according to claim 1 or the method according to claim 6, wherein the nanoparticle comprises (i) one or more metal core(s) and (ii) a SERS or a Raman label or dye surrounding the core or coated on or at the surface of the core.

12. The method according to claims 6 to 11, wherein the detection, and optionally quantification, of said complexes comprises Raman or SERS spectroscopy.

13. The kit according to claims 1 to 5 or the method according to claims 6 to 12, wherein the antibody further comprises one or more lectins and/or DNA aptamers for the detection of *Listeria.*

14. The kit according to claims 1 to 5 or the method according to claims 6 to 13, further comprising one or more non-*Listeria* specific antigen binding moiety or antigen binding fragment thereof for the detection, and optionally quantification, of bacterial species not belonging to the *Listeria* genus.

15. The kit according to claims 1 to 5 or the method according to claims 6 to 14, wherein a coating surrounds the particle or bead and the SERS or Raman label or dye; optionally, wherein the coating comprises silica, polymers, polyethylene glycol, poly(vinylpyrrolidone (PVP), polyacrylamide (PAM), poly(ethyleneimine (PEI), thioglucose and/or dextran.

## Patentansprüche

1. Sandwich-Immunoassay-Kit zur Verwendung beim Nachweisen und optional Quantifizieren von *Listerien* in einer Probe, wobei das Immunoassay-Kit Folgendes umfasst:
a) einen ersten Listerien-spezifischen Antikörper oder dessen Antigenbindungsfragment, immobilisiert auf einer ersten Oberfläche, wobei die erste Oberfläche die Oberfläche eines magnetischen Partikels, eines paramagnetischen Partikels oder eines Nanopartikels umfasst; und
b) einen zweiten Listerien-spezifischen Antikörper oder dessen Antigenbindungsfragment, immobilisiert auf einer Oberfläche eines Partikels oder Kügelchens immobilisiert ist, wobei das Partikel oder Kügelchen ferner eine SERS- oder Raman-Markierung oder einen Farbstoff umfasst,
wobei der erste und zweite Listerien-spezifische Antikörper oder dessen Antigenbindungsfragment in der Lage sind, an unterschiedliche Antigene auf der Oberfläche eines Listerien-Bakteriums zu binden, um einen nachweisbaren Komplex zu bilden, der ein zwischen dem ersten und zweiten Listerien-spezifischen Antikörpern eingeschlossenes Listerien-Bakterium umfasst, wobei der erste Listerien-spezifische Antikörper oder dessen Antigenbindungsfragment in der Lage ist, jede *Listerien-Art* zu binden, und der zweite Listerien-spezifische Antikörper oder dessen Antigenbindungsfragment in der Lage ist, eine spezifische *Listerien-Art* zu binden.

2. Kit zur Verwendung beim Nachweisen von *Listerien* nach Anspruch 1, wobei die eine oder mehreren nachzuweisenden *Listerien*-Arten ausgewählt sind aus *L. monocytogenes, L. aquatica, L. booriae, L. cornellensis, L. costaricensis, L. goaensis, L. fleischmannii, L. floridensis, L. grandensis, L. greyi, L. innocua, L. ivanovii, L. marthii, L. newyorkensis, L. riparia, L. rocourtiae, L. seeligeri, L. thailandensis, L. valentina, L. weihenstephanensis* und *L. welshimeri.*

3. Kit zur Verwendung beim Nachweisen von *Listerien* nach einem vorstehenden Anspruch, wobei die *Listerien*-Art *L. monocytogenes* umfasst und optional von einer oder mehreren weiteren *Listerien*-Arten unterschieden wird.

4. Kit zur Verwendung beim Nachweisen von *Listerien* nach einem vorstehenden Anspruch, wobei die erste Oberfläche ferner eine Beschichtungsmatrix aus einem oder mehreren Polymeren, Metall, Siliciumdioxid oder Hydroxylapatit umfasst.

5. Kit zur Verwendung beim Nachweisen von *Listerien* nach einem vorstehenden Anspruch, wobei mehr als ein zweiter Listerien-spezifischer Antikörper oder dessen Antigenbindungsfragment bereitgestellt ist, wobei jeder zweite Listerien-spezifische Antikörper oder dessen Antigenbindungsfragment für eine bestimmte Arten spezifisch ist.

6. Sandwich-Immunoassay-Verfahren zur Verwendung beim Nachweisen und optional Quantifizieren von *Listerien* in einer Probe, wobei das Immunoassay-Verfahren Folgendes umfasst:
a) Bereitstellen
i) eines ersten Listerien-spezifischen Antikörpers oder dessen Antigenbindungsfragments, immobilisiert auf einer ersten Oberfläche, wobei die erste Oberfläche die Oberfläche eines magnetischen Partikels, eines paramagnetischen Partikels oder eines Nanopartikels umfasst;
ii) eines zweiten Listerien-spezifischen Antikörpers oder dessen Antigenbindungsfragments, immobilisiert auf einer Oberfläche eines Partikels oder Kügelchens, wobei das Partikel oder Kügelchen ferner eine SERS- oder Raman-Markierung oder einen Farbstoff umfasst; und
iii) einer Flüssigkeit, die eine zu analysierende Probe umfasst;
wobei der erste und zweite Listerien-spezifische Antikörper oder deren Antigenbindungsfragmente in der Lage sind, an unterschiedliche Antigene auf der Oberfläche eines Listerien-Bakteriums zu binden, um einen nachweisbaren Komplex zu bilden, der ein zwischen dem ersten und zweiten Listerien-spezifischen Antikörpern eingeschlossenes Listerien-Bakterium umfasst, und wobei der erste Listerien-spezifische Antikörper oder dessen Antigenbindungsfragment in der Lage ist, jede *Listerien-Art* zu binden, und der zweite Listerien-spezifische Antikörper oder dessen Antigenbindungsfragmentn der Lage ist, eine spezifische *Listerien-Art* zu binden.
b) Ermöglichen, dass der erste und zweite Listerien-spezifische Antikörper oder deren Antigenbindungsfragmente und die Flüssigkeit für eine gewisse Zeit in Kontakt gebracht werden, damit sich etwaige nachweisbare Komplexe zwischen dem ersten Listerien-spezifischen Antikörper oder dessen Antigenbindungsfragment, dem zweiten Listerien-spezifischen Antikörper oder dessen Antigenbindungsfragment und etwaigen in der Flüssigkeit vorhandenen *Listerien* bilden können; und
c) Nachweisen von gebildeten Komplexen, um *Listerien* in der Probe nachzuweisen und optional zu quantifizieren.

7. Verfahren nach Anspruch 6, wobei der erste Listerien-spezifische Antikörper oder dessen Antigenbindungsfragmente auf der Oberfläche magnetischer oder paramagnetischer Partikel immobilisiert werden und etwaige Komplexe vor Nachweisen der gebildeten Komplexe mittels einer magnetischen Kraft aggregiert werden.

8. Verfahren nach den Ansprüchen 6 bis 7, wobei die nachzuweisende *Listerien-Art* ausgewählt ist aus *L. monocytogenes, L. aquatica, L. booriae, L. cornellensis, L. costaricensis, L. goaensis, L. fleischmannii, L. floridensis, L. grand ensis, L. greyi, L. innocua, L. ivanovii, L. marthii, L. newyorkensis, L. riparia, L. rocourtiae, L. seeligeri, L. thailandensis, L. valentina, L. weihenstephanensis* und *L. welshimeri.*

9. Verfahren nach den Ansprüchen 6 bis 8, wobei die *Listerien*-Art *L. monocytogenes* umfasst und optional von einer oder mehreren weiteren *Listerien*-Arten unterschieden werden kann.

10. Verfahren nach den Ansprüchen 6 bis 9, wobei die erste Oberfläche ferner eine Beschichtungsmatrix aus einem oder mehreren Polymeren, Metall, Siliciumdioxid oder Hydroxylapatit umfasst.

11. Kit nach Anspruch 1 oder Verfahren nach Anspruch 6, wobei das Nanopartikel (i) einen oder mehrere Metallkerne und (ii) eine SERS- oder Raman-Markierung oder einen Farbstoff umfasst, der den Kern umgibt oder auf oder an der Oberfläche des Kerns aufgebracht ist.

12. Verfahren nach den Ansprüchen 6 bis 11, wobei der Nachweis und optional die Quantifizierung der Komplexe Raman- oder SERS-Spektroskopie umfassen.

13. Kit nach den Ansprüchen 1 bis 5 oder Verfahren nach den Ansprüchen 6 bis 12, wobei der Antikörper ferner ein oder mehrere Lektine und/oder DNA-Aptamere zum Nachweis von *Listerien* umfasst.

14. Kit nach den Ansprüchen 1 bis 5 oder Verfahren nach den Ansprüchen 6 bis 13, ferner umfassend eine oder mehrere nicht-Listerien-spezifische Antigenbindungseinheiten oder Antigenbindungsfragmente für den Nachweis und optional die Quantifizierung von Bakterienarten, die nicht zur Gattung *Listerien* gehören.

15. Kit nach den Ansprüchen 1 bis 5 oder Verfahren nach den Ansprüchen 6 bis 14, wobei eine Beschichtung das Partikel oder Kügelchen und die SERS- oder Raman-Markierung oder den Farbstoff umgibt; wobei die Beschichtungoptional Siliciumdioxid, Polymere, Polyethylenglykol, Polyvinylpyrrolidon (PVP), Polyacrylamid (PAM), Polyethylenimin (PEI), Thioglucose und/oder Dextran umasst.

## Revendications

1. Kit de dosage immunologique en sandwich à utiliser dans la détection et facultativement la quantification de *Listeria* dans un échantillon, le kit de dosage immunologique comprenant :
a) un premier anticorps spécifique à la *Listeria,* ou un fragment de liaison à un antigène de celui-ci, immobilisé sur une première surface, dans lequel la première surface comprend la surface d'une particule magnétique, d'une particule paramagnétique ou d'une nanoparticule ; et
b) un second anticorps spécifique à la *Listeria,* ou un fragment de liaison à un antigène de celui-ci, immobilisé sur une surface d'une particule ou d'une bille, dans lequel la particule ou la bille comprend en outre un SERS ou un marqueur ou colorant Raman,
dans lequel les premier et second anticorps spécifiques à la *Listeria ,* ou leurs fragments de liaison à un antigène, sont capables de se lier à des antigènes distincts sur la surface d'une bactérie *Listeria ,* afin de former un complexe détectable comprenant une bactérie *Listeria* enserrée entre lesdits premier et second anticorps spécifiques à la *Listeria,* dans lequel le premier anticorps spécifique à la *Listeria ,* ou son fragment de liaison à un antigène, est capable de se lier à toute espèce de *Listeria* et le second anticorps spécifique à la *Listeria,* ou son fragment de liaison à un antigène, est capable de se lier à une espèce spécifique de *Listeria.*

2. Kit à utiliser pour détecter la *Listeria* selon la revendication 1, dans lequel les une ou plusieurs espèces de *Listeria* à détecter sont sélectionnées parmi *L monocytogenes, L. aquatique, L. booriae, L. cornellensis, L. costaricensis, L. goaensis, L. fleischmannii, L. floridensis, L. grandensis, L. grayi, L. innocua, L. ivanovii, L. marthii, L. newyorkensis, L. riparia, L. rocourtiae, L. seeligeri, L. thailandensis, L. valentina, L. weihenstephanensis* et *L. welshimeri.*

3. Kit à utiliser pour détecter la *Listeria* selon une quelconque revendication précédente, dans lequel l'espèce de *Listeria* comprend *L. monocytogenes* et est facultativement distinguée d'une ou plusieurs espèces de *Listeria* supplémentaires.

4. Kit à utiliser pour détecter la *Listeria* selon une quelconque revendication précédente, dans lequel la première surface comprend en outre une matrice de revêtement d'un ou plusieurs polymères, de métal, de silice ou d'hydroxyapatite.

5. Kit à utiliser pour détecter la *Listeria* selon une quelconque revendication précédente, dans lequel plus d'un second anticorps spécifique à la *Listeria,* ou fragment de liaison à un antigène de celui-ci, est fourni, dans lequel chaque second anticorps spécifique à la *Listeria,* ou fragment de liaison à l'antigène de celui-ci, est spécifique pour une espèce particulière.

6. Procédé de dosage immunologique en sandwich à utiliser dans la détection et facultativement la quantification de *Listeria* dans un échantillon, ledit procédé de dosage immunologique comprenant :
a) la fourniture
i) d'un premier anticorps spécifique à la *Listeria ,* ou d'un fragment de liaison à un antigène de celui-ci, immobilisé sur une première surface, dans lequel la première surface comprend la surface d'une particule magnétique, d'une particule paramagnétique ou d'une nanoparticule ;
ii) d'un second anticorps spécifique à la *Listeria ,* ou d'un fragment de liaison à un antigène de celui-ci, immobilisé sur une surface d'une particule ou d'une bille, dans lequel la particule ou la bille comprend en outre un SERS ou un marqueur ou colorant Raman ; et
iii) d'un liquide comprenant un échantillon à analyser ;
dans lequel les premier et second anticorps spécifiques à la *Listeria,* ou leurs fragments de liaison à un antigène, sont capables de se lier à des antigènes distincts sur la surface d'une bactérie *Listeria,* afin de former un complexe détectable comprenant une bactérie *Listeria* enserrée entre lesdits premier et second anticorps spécifiques à la *Listeria ,* dans lequel le premier anticorps spécifique à la *Listeria,* ou son fragment de liaison à un antigène, est capable de se lier à toute espèce de *Listeria* et le second anticorps spécifique à la *Listeria,* ou son fragment de liaison à un antigène, est capable de se lier à une espèce spécifique de *Listeria.*
b ) la permission aux premier et second anticorps spécifiques à la *Listeria,* ou à leurs fragments de liaison à un antigène, d'entrer en contact avec le liquide pendant une période déterminée, afin que de quelconques complexes détectables puissent se former entre le premier anticorps spécifique à la *Listeria,* ou son fragment de liaison à un antigène, le deuxième anticorps spécifique *de Listeria,* ou son fragment de liaison à un antigène, et toute *Listeria* présente dans le liquide ; et
c) la détection de quelconques complexes qui se forment, afin de détecter et facultativement de quantifier toute *Listeria* dans l'échantillon.

7. Procédé selon la revendication 6, dans lequel le premier anticorps spécifique à la *Listeria,* ou des fragments de liaison à un antigène de celui-ci, sont immobilisés à la surface de particules magnétiques ou paramagnétiques, et de quelconques complexes sont agrégés à l'aide d'une force magnétique avant de détecter de quelconques complexes qui sont formés.

8. Procédé selon les revendications 6 à 7, dans lequel l'espèce de *Listeria* à détecter est sélectionnée parmi *L. monocytogenes, L. Aquatica, L. booriae, L. cornellensis, L. costoricensis, L. goaensis, L. fleischmannii, L. floridensis, L. grand ensis, L. grayi, L. innocua, L. ivanovii, L. marthii, L. newyorkensis, L. riparia, L. rocourtiae, L. seeligeri, L. thoilondensis, L. valentina, L. weihenstephanensis etL. welshimeri.*

9. Procédé selon les revendications 6 à 8, dans lequel l'espèce de *Listeria* comprend *L. monocytogenes* et est facultativement distinguée d'une ou plusieurs espèces de *Listeria* supplémentaires.

10. Procédé selon les revendications 6 à 9, dans lequel la première surface comprend en outre une matrice de revêtement d'un ou plusieurs polymères, de métal, de silice ou d'hydroxyapatite.

11. Kit selon la revendication 1 ou procédé selon la revendication 6, dans lequel la nanoparticule comprend (i) un ou plusieurs noyaux métalliques et (ii) un SERS ou un marqueur ou colorant Raman entourant le noyau ou appliqué en revêtement sur ou à la surface du noyau.

12. Procédé selon les revendications 6 à 11, dans lequel la détection, et facultativement la quantification, desdits complexes comprend une spectroscopie Raman ou SERS.

13. Kit selon les revendications 1 à 5 ou procédé selon les revendications 6 à 12, dans lequel l'anticorps comprend en outre une ou plusieurs lectines et/ou un ou plusieurs aptamères d'ADN pour la détection de *Listeria.*

14. Kit selon les revendications 1 à 5 ou procédé selon les revendications 6 à 13, comprenant en outre une ou plusieurs fractions de liaison à un antigène non spécifiques à la *Listeria* ou un fragment de liaison à un antigène de celles-ci pour la détection, et facultativement la quantification, d'espèces bactériennes n'appartenant pas au genre *Listeria.*

15. Kit selon les revendications 1 à 5 ou procédé selon les revendications 6 à 14, dans lequel un revêtement entoure la particule ou la bille et le SRS ou marqueur ou colorant Raman ; facultativement dans lequel le revêtement comprend de la silice, des polymères, du polyéthylène glycol, de la polyvinylpyrrolidone (PVP), du polyacrylamide (PAM), de la poly(éthylèneimine) (PEI), du thioglucose et/ou du dextrane.
